(19) Europäisches Patentamt
European Patent Office
Office européen des brevets

(11) EP 1 142 883 A1

(12) **EUROPEAN PATENT APPLICATION**
published in accordance with Art. 158(3) EPC

(43) Date of publication:
**10.10.2001 Bulletin 2001/41**

(21) Application number: **99973410.6**

(22) Date of filing: **02.12.1999**

(51) Int Cl.7: **C07D 263/54**, C07D 413/12, A61K 31/42, A61K 31/423, A61K 31/538, A61K 31/553

(86) International application number:
**PCT/JP99/06758**

(87) International publication number:
**WO 00/35890 (22.06.2000 Gazette 2000/25)**

(84) Designated Contracting States:
**AT BE CH CY DE DK ES FI FR GB GR IE IT LI LU MC NL PT SE**
Designated Extension States:
**AL LT LV MK RO SI**

(30) Priority: **15.12.1998 JP 35574198**

(71) Applicant: **Asahi Kasei Kabushiki Kaisha**
**Osaka-shi, Osaka 530-8205 (JP)**

(72) Inventors:
- **MIYOSHI, Shiro**
  **Fuji-shi Shizuoka 416-0946 (JP)**
- **OGAWA, Kohei**
  **Mishima-shi Shizuoka 411-0802 (JP)**

(74) Representative:
**Forstmeyer, Dietmar, Dr. rer. nat., Dipl.-Chem. et al**
**Boeters & Bauer,**
**Bereiteranger 15**
**81541 München (DE)**

(54) **NOVEL HETEROCYCLIC COMPOUNDS AND DRUG COMPOSITIONS CONTAINING THE SAME**

(57) The present invention is directed to a compound of the general formula (I):

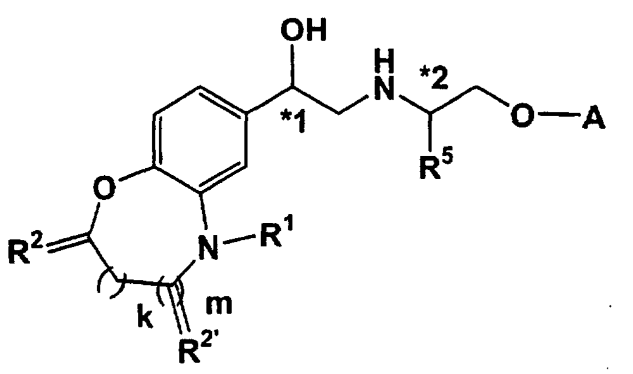

(I)

wherein $R^1$ represents a hydrogen atom, a methyl group or $SO_2R^3$; $R^2$ represents O, S or $H_2$; $R^{2'}$ represents O or $H_2$; $R^3$ represents a lower alkyl group or $NH^4R^{4'}$; $R^4$ and $R^{4'}$ may be the same or different and represent a hydrogen atom, a lower alkyl group or a benzyl group; $R^5$ represents a hydrogen atom or a lower alkyl group; k and m are zero or 1; A represents the general formula (II) or (III):

EP 1 142 883 A1

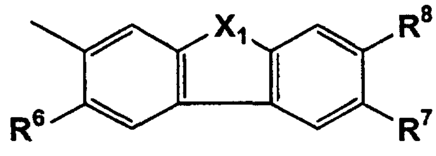 (II) 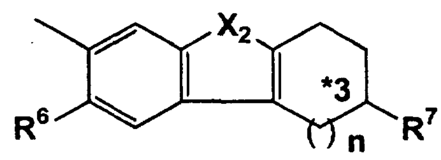 (III)

$X_1$ represents a secondary nitrogen atom, an oxygen atom, a sulfur atom or a methylene group; n is 1 or 2; $X_2$ represents a secondary nitrogen atom, an oxygen atom or a sulfur atom; and, $R^6$, $R^7$ and $R^8$ are a hydrogen atom or the like; and a salt thereof. The compound of the present invention provides a pharmaceutical composition for treating and preventing β3-associated diseases, such as diabetes, obesity and hyperlipidemia. The composition is particularly suitable for oral administration.

## Description

FIELD OF THE INVENTION

This invention relates to novel heterocyclic compounds and pharmaceutical compositions containing the same.

BACKGROUND OF THE INVENTION

In the past, β-adrenoreceptors were classified into two classes, β1-adrenoreceptor and β2-adrenoreceptor, and it was recognized that stimulation of β1 induces an increase of the heart rate and stimulation of β2 induces a relaxation of the smooth muscle tissue, both resulting in lowering the blood pressure. Arch et al. showed the presence of the third receptor by finding a compound which has very little effects on β1 and β2 and facilitates the lipolysis of fat cells (Nature, 309, pp. 163-165 (1984)). Then, the primary structure of the third receptor was elucidated (Emorine et al., Science, 245, pp. 1118-1121 (1989)), and it was named as β3.

Recently, compounds having β3-agonist activity were shown to be useful as a medicine for treating and preventing diabetes, obesity, hyperlipidemia, digestive diseases and depression (Int. J. Obesity, 8 (Suppl. 1), pp. 93-102 (1984); Nature, 309, pp. 163-165 (1984); USP 5,120,766; Brit. J. Pharmacol., 103, pp. 1351-1356 (1991); Eur. J. Pharmacol., 219, pp. 193-201 (1992)).

So far, examples of compounds relating to β3 have included the following compounds:

the compound (BRL 37344) having the following structural formula described in EP 023385 and Drugs of the future, 16, pp. 797-800 (1991):

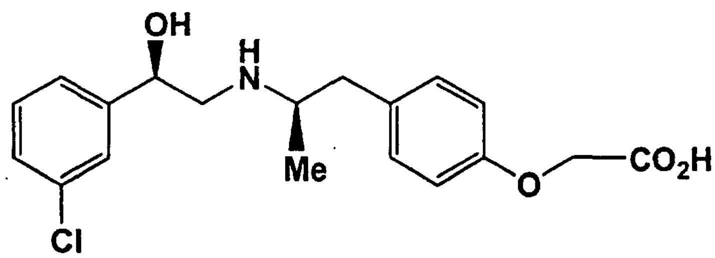

the compound (CL 316,243) having the following structural formula described in EP 0455006 and J. Med. Chem., 35, pp. 3081-3084 (1992):

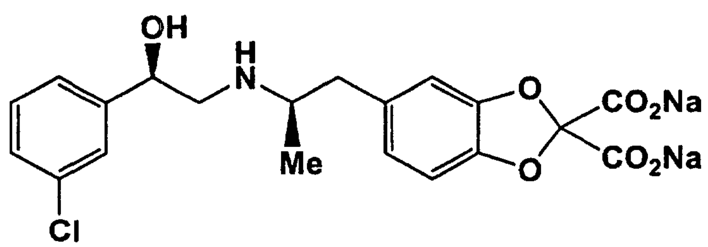

and

the compound having the following structural formula described in WO 94/29290:

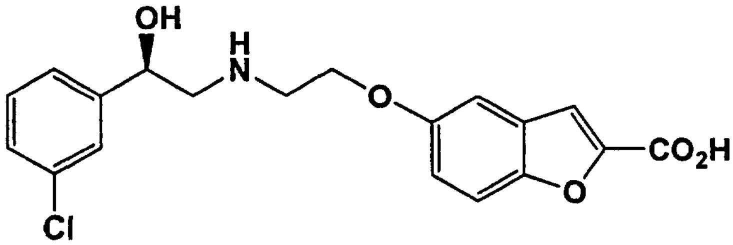

Further, EP 0659737 discloses a variety of compounds and specifically describes as an example in Example 1 in the text of specification the compound having the following structural formula:

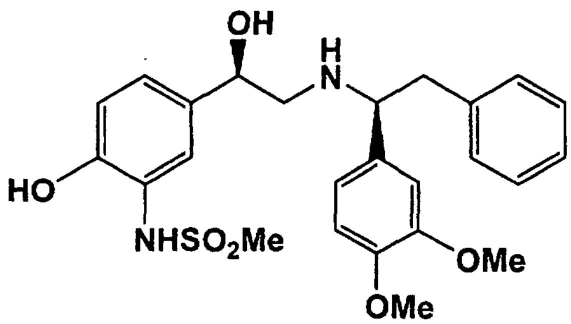

WO 96/35685 describes the compound having the following structural formula:

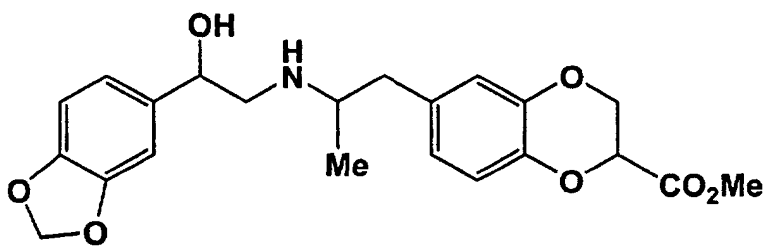

However, the chemical structures of the above compounds are apparently different from those of the claimed compounds of the present invention.

In addition, the compound having heart rate-increasing activity, myocardial contraction enhancement and antiobestic activity, which has the following structural formula:

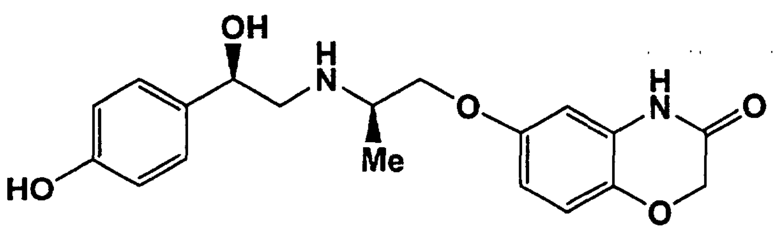

described in EP 171702 is known. However, this compound acts on the heart and is different from the compound of the present invention in the chemical structure and in that the former strongly acts on the heart.

Further, the compound having $\alpha,\beta$-blocking or hypotensive activity, which has the following structural formula:

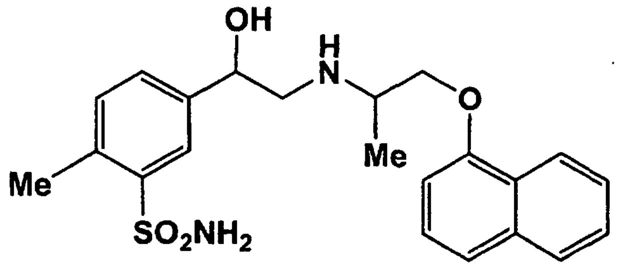

described in JP-A-55-53262 and JP-A-58-41860 is known; and the compound having vasodilator action, which has the following structural formula:

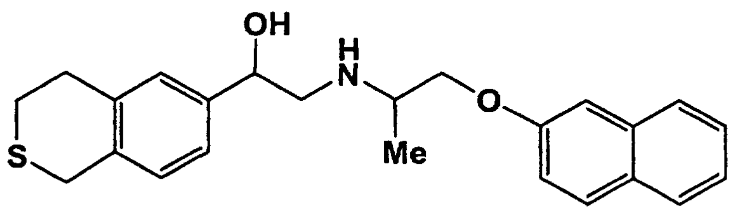

described in DE 2651572 is known. However, these compounds are different from the compounds of the present invention in their chemical structures and intended uses.

Further, USP 4,816,457 describes the compound which contains a benzoxazine ring and is represented by the following structural formula:

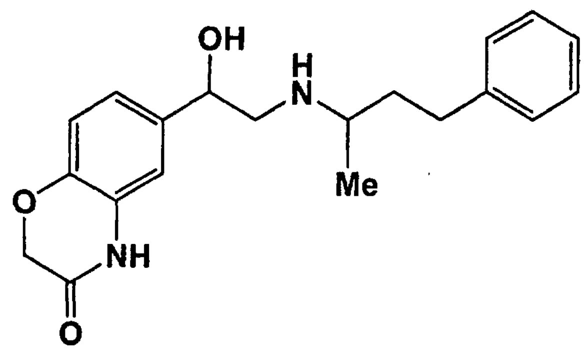

and DE 2429253, which discloses a variety of compounds, specifically describes as an example in Example 28 in the text of specification the compound having the following structural formula:

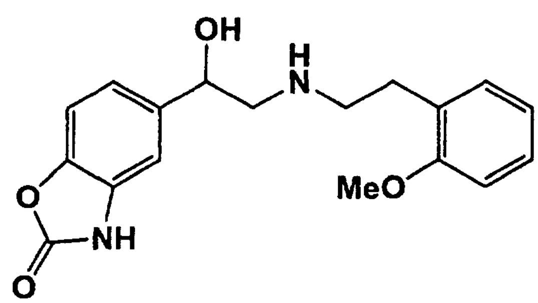

However, these compounds have α,β-blocking activity and are different from the compounds of the present invention in their chemical structures and intended uses.

The present inventors formerly invented compounds having excellent β3-agonist activity and disclosed compounds represented by, for example, the following structural formula in WO 97/25311.

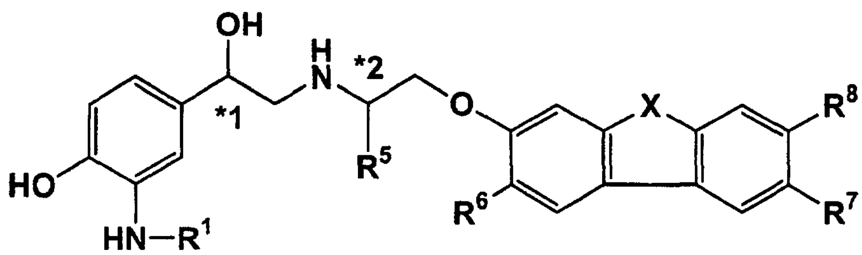

PROBLEMS TO BE SOLVED

There has been a need for a novel and useful medicine for treating and preventing β3-associated diseases, such as diabetes, obesity and hyperlipidemia.

MEANS TO SOLVE THE PROBLEMS

In order to solve the above problems, the present inventors synthesized a variety of compounds and investigated their activities. As a result, the invention disclosed in WO 97/25311 mentioned above was completed. However, further evaluation of said compounds by the present inventors showed that some of said compounds did not necessarily provide a desired blood concentration via oral administration. Therefore, it was deemed that there were needs to provide more useful compounds. The present inventors earnestly continued such investigations and further synthesized a great many compounds. As a result, the present inventors have found that a novel heterocyclic compound of the general formula (I) as set forth below has β3-agonist activity; has excellent permeability through human small intestine epithelium when orally administered; is expected to provide a desired blood concentration; and can exhibit adequate hypoglycemic activity and lipolytic activity when orally administered, and completed the present invention.

That is, the first aspect of the present invention is a compound of the general formula (I):

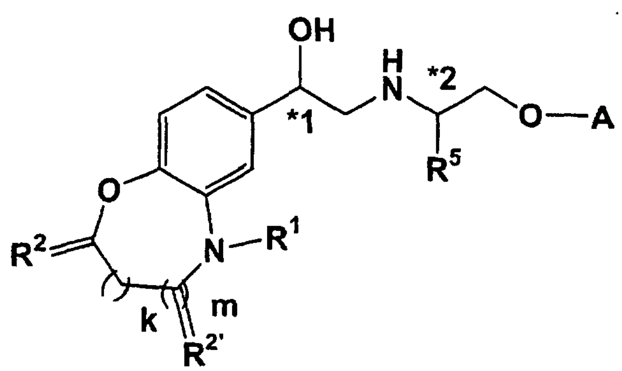

(I)

or a salt thereof,
wherein

$R^1$ represents a hydrogen atom, a methyl group or $SO_2R^3$;
$R^2$ represents O, S or $H_2$;
$R^{2'}$ represents O or $H_2$;
$R^3$ represents a lower alkyl group or $NR^4R^{4'}$;
$R^4$ and $R^{4'}$ may be the same or different and represent a hydrogen atom, a lower alkyl group or a benzyl group;
$R^5$ represents a hydrogen atom or a lower alkyl group;
k and m are zero or 1;
A represents the general formula (II) or (III):

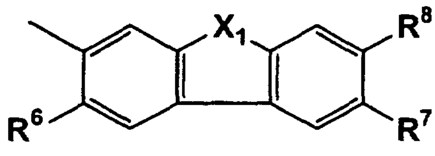 (II) 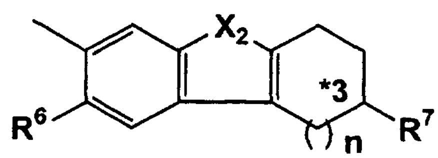 (III)

when A represents the general formula (II),
$X_1$ represents a secondary nitrogen atom, an oxygen atom, a sulfur atom or a methylene group; and
when $X_1$ represents a secondary nitrogen, oxygen or sulfur atom, then $R^8$ represents a hydrogen atom, one of $R^6$ and $R^7$ represents a hydrogen atom, and the other represents a hydrogen atom, an amino group, an acetylamino group or a hydroxyl group; or
when $X_1$ is a methylene group, then $R^6$ and $R^7$ both represent a hydrogen atom and $R^8$ represents a hydrogen atom, an amino group, an acetylamino group or a hydroxyl group; or
when A represents the general formula (III),
n is 1 or 2;
$X_2$ represents a secondary nitrogen atom, an oxygen atom or a sulfur atom;
when n is 1, then one of $R^6$ and $R^7$ represents a hydrogen atom, and the other represents a hydrogen atom, an amino group, an acetylamino group or a hydroxyl group; or
when n is 2, then $R^7$ represents a hydrogen atom, and $R^6$ represents a hydrogen atom, an amino group, an acetylamino group or a hydroxyl group;

- *1 represents an asymmetric carbon atom;
- *2 represents an asymmetric carbon atom when $R^5$ is a lower alkyl group; and
- *3 represents an asymmetric carbon atom when $R^7$ is an amino, acetylamino or hydroxyl group.

The second aspect of the present invention is a compound of the general formula (I), wherein A represents the general formula (II), and $R^1$, $R^2$, $R^{2'}$, $R^5$, k, m, $R^6$, $R^7$, $R^8$, *1 and *2 have the same meanings as defined above, or a salt thereof

The third aspect of the present invention is a compound of the general formula (I), wherein A represents the general formula (III), and $R^1$, $R^2$, $R^{2'}$, $R^5$, k, m, n, $R^6$, $R^7$, *1, *2 and *3 have the same meanings as defined above, or a salt thereof.

As used herein, "lower alkyl" means a straight or branched saturated hydrocarbon containing 1 to 4 carbon atoms and includes methyl, ethyl, n-propyl, i-propyl, n-butyl, i-butyl, sec-butyl and tert-butyl.

$R^1$ is a hydrogen atom, a methyl group or $SO_2R^3$ and the compounds of the general formula (I) wherein $R^1$ is $SO_2R^3$ are preferred. $R^3$ of $SO_2R^3$ is a lower alkyl group or $NR^4R^{4'}$, and $R^4$ and $R^{4'}$ may be the same or different and represent a hydrogen atom, a lower alkyl group or a benzyl group. Specifically, $NR^4R^{4'}$ may be amino, methylamino, ethylamino, propylamino, benzylamino, dimethylamino, diethylamino, dipropylamino, methylethylamino, methylpropylamino or methylbenzylamino, with a dimethylamino group being more preferred. In consequence, preferred specific examples of $SO_2R^3$ include $SO_2Me$, $SO_2Et$, $SO_2CH_2Ph$, $SO_2NH_2$, $SO_2NHMe$, $SO_2NHEt$, $SO_2NMe_2$, $SO_2NEt_2$, $SO_2NMeEt$ and $SO_2NMeCH_2Ph$.

$R^2$ is O, S or $H_2$ (two hydrogen atoms), $R^{2'}$ is O or $H_2$, and k and m each are zero or 1. When k or m is zero, the corresponding moiety means a single bond. Within the combinations of the above variables, the combination in which $R^2$ is O or S and k and m both are zero is preferred. The combination in which $R^2$ and $R^{2'}$ both are $H_2$ and either k or m is zero is also preferred. A compound of the general formula (I) in which k is 1 and m is zero can be substantially the same with a compound in which k is zero, m is 1 and $R^{2'}$ is $H_2$. When it is needed to distinguish the above two cases, the former is preferably adopted.

$R^5$ represents a hydrogen atom or a lower alkyl group. Preferred examples of $R^5$ include hydrogen, methyl and ethyl. $R^5$ is more preferably a hydrogen atom.

In the general formula (II), $X_1$ is a secondary nitrogen atom, an oxygen atom, a sulfur atom or a methylene group, with a secondary nitrogen atom being preferred. $R^6$, $R^7$ and $R^8$ are as defined above.

In the general formula (III), $X_2$ is a secondary nitrogen atom, an oxygen atom or a sulfur atom. Preferred compounds include those of the general formula (III) in which $X_2$ is a secondary nitrogen atom and n is 1 (that is, the compounds having a skeleton of tetrahydrocarbazole as the tricyclic group). $R^6$ and $R^7$ are as defined above.

When $R^7$ of the general formula (III) is a hydrogen atom, *1 of the general formula (I) is an asymmetric carbon atom, and in case $R^5$ is a lower alkyl group, *2 is also an asymmetric carbon atom. In this case, the compound of the general formula (I) can be in the form of any of four different isomers, i.e. (R, R), (R, S), (S, S) and (S, R) (indicated in

order of (*1, *2)). When $R^5$ is a hydrogen atom, two different isomers exist. Not only optically pure isomers, but also a mixture of any two isomers, a mixture of any three isomers and a mixture of all the four isomers are encompassed in the present invention. From the viewpoint of pharmacological activity, a preferred configuration of the asymmetric carbon (*1) in the ethanolamino chain may be the absolute configuration R. With respect to the asymmetric carbon (*1) of 6-[2-[2-(9H-carbazol-2-yloxy)ethylamino]-1-hydroxyethyl]-4-(methylsulfonyl)-2,3-dihydro-1,4-benzoxazine, R-hydroxyl structure may be particularly preferred.

Further, when $R^7$ of the general formula (III) is not a hydrogen atom, in the general formula (I), *3 and *1 are an asymmetric carbon atom, and in case $R^5$ is a lower alkyl group, *2 is also an asymmetric carbon atom. In this case, there are three asymmetric carbon atoms at most and the compound of the general formula (I) can be in the form of any of eight different isomers. Not only optically pure isomers, but also any mixture of isomers are included in the present invention. From the viewpoint of pharmacological activity, a preferred configuration of the asymmetric carbon (*1) in the ethanolamino chain is the absolute configuration R. With respect to the asymmetric carbon atom *3, both optically active form and racemic form are preferred.

According to the present invention, a variety of combinations of the substituents can form some very preferred classes of the claimed compounds. $R^1$, $R^5$, $X_1$, $X_2$, n, $R^6$, $R^7$, $R^8$, *1, *2 and *3 are as defined above for the first to third aspects of the present invention, unless otherwise specified.

According to the present invention, compounds of the general formula (I) in which $R^2$ and $R^{2'}$ represent $H_2$, and k and m represent zero or 1, or salts thereof, may be mentioned as preferred examples.

Further, compounds of the general formula (I) in which $R^2$ represents O or S, and k and m represent zero, or salts thereof, may be also mentioned as preferred examples.

Compounds of the general formula (I) in which $R^2$ represents O, $R^{2'}$ represents O or $H_2$, k is zero, and m is 1, or salts thereof, may be also mentioned as preferred examples.

Compounds of the general formula (I) in which $R^2$ represents $H_2$, $R^{2'}$ represents O, k is zero, and m is 1, or salts thereof, may be also mentioned as preferred examples.

In addition, the following compounds may be mentioned as specific compounds of the general formula (I) of the present invention in which A represents the general formula (II).

(R)-2-[2-(9H-carbazol-2-yloxy)ethylamino]-1-(2,3-dihydrobenzoxazol-5-yl)ethan-1-ol;
(R)-5-[2-[2-(9H-carbazol-2-yloxy)ethylamino]-1-hydroxyethyl]-3-(methylsulfonyl)-2,3-dihydrobenzoxazole;
(S)-5-[2-[2-(9H-carbazol-2-yloxy)ethylamino]-1-hydroxyethyl]-3-(methylsulfonyl)-2,3-dihydrobenzoxazole;
5-[2-[2-(9H-carbazol-2-yloxy)ethylamino]-1-hydroxyethyl]-3-(methylsulfonyl)-2,3-dihydrobenzoxazole;
(R)-5-[2-[2-(dibenzothiophen-3-yloxy)ethylamino]-1-hydroxyethyl]-3-(methylsulfonyl)-2,3-dihydrobenzoxazole;
(R)-5-[2-[2-(dibenzofuran-3-yloxy)ethylamino]-1-hydroxyethyl]-3-(methylsulfonyl)-2,3-dihydrobenzoxazole;
(R)-5-[2-[2-(7-acetylaminofluoren-2-yloxy)ethylamino]-1-hydroxyethyl]-3-(methylsulfonyl)-2,3-dihydrobenzoxazole;
(R)-5-[2-[2-(7-aminofluoren-2-yloxy)ethylamino]-1-hydroxyethyl]-3-(methylsulfonyl)-2,3-dihydrobenzoxazole;
(R)-[5-[2-[2-(9H-carbazol-2-yloxy)ethylamino]-1-hydroxyethyl]-(2,3-dihydrobenzoxazol-3-yl)sulfonyl]dimethylamine:
(R)-[5-[2-[2-(dibenzofuran-3-yloxy)ethylamino]-1-hydroxyethyl]-(2,3-dihydrobenzoxazol-3-yl)sulfonyl]dimethylamine;
(R)-[5-[2-[2-(7-acetylaminofluoren-2-yloxy)ethylamino]-1-hydroxyethyl]-(2,3-dihydrobenzoxazol-3-yl)sulfonyl] dimethylamine;
(R)-[5-[2-[2-(7-aminofluoren-2-yloxy)ethylamino]-1-hydroxyethyl]-(2,3-dihydrobenzoxazol-3-yl)sulfonyl]dimethylamine;
(R)-1-[(4H-2,3-dihydro-1,4-benzoxazin)-6-yl]-2-[2-(9H-carbazol-2-yloxy)ethylamino]ethan-1-ol;
(R)-1-[(4-methyl-2,3-dihydro-1,4-benzoxazin)-6-yl]-2-[2-(9H-carbazol-2-yloxy)ethylamino]ethan-1-ol;
(R)-6-[2-[2-(9H-carbazol-2-yloxy)ethylamino]-1-hydroxyethyl]-4-(methylsulfonyl)-2,3-dihydro-1,4-benzoxazine;
(R)-6-[2-[2-(dibenzothiophen-3-yloxy)ethylamino]-1-hydroxyethyl]-4-(methylsulfonyl)-2,3-dihydro-1,4-benzoxazine;
(R)-6-[2-[2-(dibenzofuran-3-yloxy)ethylamino]-1-hydroxyethyl]-4-(methylsulfonyl)-2,3-dihydro-1,4-benzoxazine;
(R)-6-[2-[2-(7-acetylaminofluoren-2-yloxy)ethylamino]-1-hydroxyethyl]-4-(methylsulfonyl)-2,3-dihydro-1,4-benzoxazine;
(R)-6-[2-[2-(7-aminofluoren-2-yloxy)ethylamino]-1-hydroxyethyl]-4-(methylsulfonyl)-2,3-dihydro-1,4-benzoxazine;
(R)-[[6-[2-[2-(9H-carbazol-2-yloxy)ethylamino]-1-hydroxyethyl]-(2,3-dihydro-1,4-benzoxazin)-4-yl]sulfonyl] dimethylamine;
(R)-[[6-[2-[2-(dibenzothiophen-3-yloxy)ethylamino]-1-hydroxyethyl]-(2,3-dihydro-1,4-benzoxazin)-4-yl]sulfonyl]-dimethylamine;

(R)-[[6-[2-[2-(dibenzofuran-3-yloxy)ethylamino]-1-hydroxyethyl]-(2,3-dihydro-1,4-benzoxazin)-4-yl]sulfonyl] dimethylamine;
(R)-[[6-[2-[2-(7-acetylaminofluoren-2-yloxy)ethylamino]-1-hydroxyethyl]-(2,3-dihydro-1,4-benzoxazin)-4-yl]sulfonyl]-dimethylamine;
(R)-[[6-[2-[2-(7-aminofluoren-2-yloxy)ethylamino]-1-hydroxyethyl]-(2,3-dihydro-1,4-benzoxazin)-4-yl]sulfonyl]-dimethylamine;
(R)-7-[2-[2-(9H-carbazol-2-yloxy)ethylamino]-1-hydroxyethyl]-5-(methylsulfonyl)-2H,3H,4H-benzo[b]1,4-oxazepine;
(R)-7-[2-[2-(dibenzothiophen-3-yloxy)ethylamino]-1-hydroxyethyl]-5-(methylsulfonyl)-2H,3H,4H-benzo[b]1,4-oxazepine;
(R)-7-[2-[2-(dibenzofuran-3-yloxy)ethylamino]-1-hydroxyethyl]-5-(methylsulfonyl)-2H,3H,4H-benzo[b]1,4-oxazepine;
(R)-[[7-[2-[2-(9H-carbazol-2-yloxy)ethylamino]-1-hydroxyethyl]-(2H,3H,4H-benzo[b]1,4-oxazepin-5-yl)]sulfonyl] dimethylamine;
(R)-5-[2-[2-(9H-carbazol-2-yloxy)ethylamino]-1-hydroxyethyl]-3-(methylsulfonyl)-3-hydrobenzoxazol-2-one;
(S)-5-[2-[2-(9H-carbazol-2-yloxy)ethylamino]-1-hydroxyethyl]-3-(methylsulfonyl)-3-hydrobenzoxazol-2-one;
5-[2-[2-(9H-carbazol-2-yloxy)ethylamino]-1-hydroxyethyl]-3-(methylsulfonyl)-3-hydrobenzoxazol-2-one;
(R)-5-[2-[2-(9H-carbazol-2-yloxy)ethylamino]-1-hydroxyethyl]-3-(methylsulfonyl)-3-hydrobenzoxazol-2-thione;
(R) -3-[(dimethylamino)sulfonyl]-5-[2-[2-(9H-carbazol-2-yloxy)ethylamino]-1-hydroxyethyl]-3-hydrobenzoxazol-2-one;
(S)-3-[(dimethylamino)sulfonyl]-5-[2-[2-(9H-carbazol-2-yloxy)ethylamino]-1-hydroxyethyl]-3-hydrobenzoxazol-2-one;
3-[(dimethylamino)sulfonyl]-5-[2-[2-(9H-carbazol-2-yloxy)ethylamino]-1-hydroxyethyl]-3-hydrobenzoxazol-2-one;
(R)-5-[2-[2-(9H-carbazol-2-yloxy)ethylamino]-1-hydroxyethyl]-3-hydrobenzoxazol-2-one;
(R)-5-[2-[2-(9H-carbazol-2-yloxy)ethylamino]-1-hydroxyethyl]-3-methyl-3-hydrobenzoxazol-2-one;
(R)-6-[2-[2-(9H-carbazol-2-yloxy)ethylamino]-1-hydroxyethyl]-2H-1,4-benzoxazin-3(4H)-one;
(R)-6-[2-[2-(9H-carbazol-2-yloxy)ethylamino]-1-hydroxyethyl]-4-methyl-2H-1,4-benzoxazin-3-one;
(R)-6-[2-[2-(9H-carbazol-2-yloxy)ethylamino]-1-hydroxyethyl]-4-(methylsulfonyl)-2H-1,4-benzoxazin-3-one;
(R)-4-[(dimethylamino)sulfonyl]-6-[2-[2-(9H-carbazol-2-yloxy)ethylamino]-1-hydroxyethyl]-2H-1,4-benzoxazin-3-one;
(R)-6-[2-[2-(9H-carbazol-2-yloxy)ethylamino]-1-hydroxyethyl]-3H-1,4-benzoxazin-2(4H)-one;
(R)-6-[2-[2-(9H-carbazol-2-yloxy)ethylamino]-1-hydroxyethyl]-4-methyl-3H-1,4-benzoxazin-2-one;
(R)-6-[2-[2-(9H-carbazol-2-yloxy)ethylamino]-1-hydroxyethyl]-4-(methylsulfonyl)-3H-1,4-benzoxazin-2-one; and
(R)-4-[(dimethylamino)sulfonyl]-6-[2-[2-(9H-carbazol-2-yloxy)ethylamino]-1-hydroxyethyl]-3H-1,4-benzoxazin-2-one.

Further, the following compounds may be mentioned as specific compounds of the general formula (I) of the present invention in which A represents the general formula (III).

(R)-5-[2-[2-(5,6,7,8-tetrahydro-9H-carbazol-2-yloxy)ethylamino]-1-hydroxyethyl]-3-(methylsulfonyl)-2,3-dihydrobenzoxazole;
(R) -5- [2- [2- (6,7,8,9-tetrahydrodibenzothiophen-3-yloxy)-ethylamino]-1-hydroxyethyl]-3-(methylsulfonyl)-2,3-dihydrobenzoxazole;
(R)-5-[2-[2-(5,6,7,8,9,10-hexahydro-cyclohepta[b]indol-2-yloxy)ethylamino]-1-hydroxyethyl]-3-(methylsulfonyl)-2,3-dihydrobenzoxazole;
(R)-[5-[2-[2-(5,6,7,8-tetrahydro-9H-carbazol-2-yloxy)-ethylamino]-1-hydroxyethyl]-(2,3-dihydrobenzoxazol-3-yl) sulfonyl]-dimethylamine;
(R)-[5-[2-[2-(5,6,7,8,9,10-hexahydro-cyclohepta[b]indol-2-yloxy)ethylamino]-1-hydroxyethyl]-(2,3-dihydrobenzoxazol-3-yl)sulfonyl]dimethylamine;
(R)-6-[2-[2-(5,6,7,8-tetrahydro-9H-carbazol-2-yloxy)ethylamino]-1-hydroxyethyl]-4-(methylsulfonyl)-2,3-dihydro-1,4-benzoxazine;
(R)-6-[2-[2-(5,6,7,8,9,10-hexahydro-cyclohepta[b]indol-2-yloxy)ethylamino]-1-hydroxyethyl]-4-(methylsulfonyl)-2,3-dihydro-1,4-benzoxazine;
(R)-[[6-[2-[2-(5,6,7,8-tetrahydrocarbazol-2-yloxy)ethylamino]-1-hydroxyethyl]-(2,3-dihydro-1,4-benzoxazin)-4-yl] sulfonyl]-dimethylamine;
(R)-[[6-[2-[2-(5,6,7,8,9,10-hexahydro-cyclohepta[b]indol-2-yloxy)ethylamino]-1-hydroxyethyl]-(2,3-dihydro-1,4-benzoxazin)-4-yl]sulfonyl]dimethylamine;
(R)-7-[2-[2-(5,6,7,8-tetrahydro-9H-carbazol-2-yloxy)ethylamino]-1-hydroxyethyl]-5-(methylsulfonyl)-2H,3H,4H-benzo[b]1,4-oxazepine;
(R)-[[7-[2-[2-(5,6,7,8-tetrahydro-9H-carbazol-2-yloxy)-ethylamino]-1-hydroxyethyl](2H,3H,4H-benzo[b]1,4-oxazepin-5-yl)]sulfonyl]dimethylamine;
(R)-6-[2-[2-(5,6,7,8-tetrahydro-9H-carbazol-2-yloxy)-ethylamino]-1-hydroxyethyl]-4-(methylsulfonyl)-2H-1,4-benzoxazin-3-one;
(R)-4-[(dimethylamino)sulfonyl]-6-[2-[2-(5,6,7,8-tetrahydro-9H-carbazol-2-yloxy)ethylamino]-1-hydroxyethyl]-2H-1,4-benzoxazin-3-one;
(R)-6-[2-[2-(5,6,7,8-tetrahydro-9H-carbazol-2-yloxy)-ethylamino]-1-hydroxyethyl]-4-(methylsulfonyl)-3H-1,4-benzoxazin-2-one; and
(R)-4-[(dimethylamino)sulfonyl]-6-[2-[2-(5,6,7,8-tetrahydro-9H-carbazol-2-yloxy)ethylamino]-1-hydroxyethyl]-3H-1,4-benzoxazin-2-one.

The compounds of the general formula (I) can be prepared by, for example, the following process.

[Process for the preparation]

A compound of the general formula (IV):

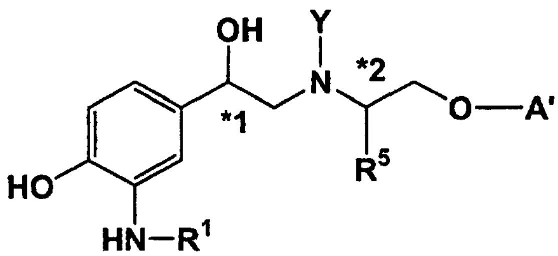

(IV)

wherein

$R^1$ represents a hydrogen atom, a methyl group or $SO_2R^3$;
$R^3$ represents a lower alkyl group or $NR^4R^{4'}$;
$R^4$ and $R^{4'}$ may be the same or different and represent a hydrogen atom, a lower alkyl group or a benzyl group;
$R^5$ represents a hydrogen atom or a lower alkyl group;
Y represents an amine-protecting group;
A' represents the general formula (II') or (III'):

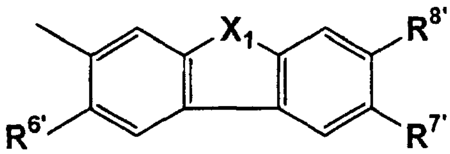

(II')

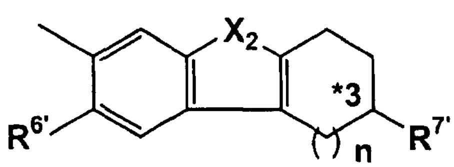

(III')

when A' represents the general formula (II'),
$X_1$ represents a secondary nitrogen atom, an oxygen atom, a sulfur atom or a methylene group; and
when $X_1$ represents a secondary nitrogen, oxygen or sulfur atom, then $R^{8'}$ represents a hydrogen atom, one of $R^{6'}$ and $R^{7'}$ represents a hydrogen atom, and the other represents a hydrogen atom, an acetylamino group or a hydroxyl group protected with a protecting group C; or
when $X_1$ is a methylene group, then $R^{6'}$ and $R^{7'}$ both represent a hydrogen atom and $R^{8'}$ represents a hydrogen atom, an acetylamino group or a hydroxyl group protected with a protecting group C; or when A' represents the general formula (III'),
n is 1 or 2;
$X_2$ represents a secondary nitrogen atom, an oxygen atom or a sulfur atom; and
when n is 1, then one of $R^{6'}$ and $R^{7'}$ represents a hydrogen atom, and the other represents a hydrogen atom, an acetylamino group or a hydroxyl group protected with a protecting group C; or

when n is 2, then $R^{7'}$ represents a hydrogen atom, and $R^{6'}$ represents a hydrogen atom, an acetylamino group or a hydroxyl group protected with a protecting group C;

* 1 represents an asymmetric carbon atom;
* 2 represents an asymmetric carbon atom when $R^5$ is a lower alkyl group; and
* 3 represents an asymmetric carbon atom when $R^{7'}$ is an acetylamino group or a hydroxyl group protected with a protecting group C,
 is reacted with a compound of the general formula (V):

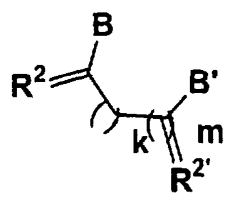

(V)

wherein $R^2$ represents O, S or $H_2$; $R^{2'}$ represents O or $H_2$; k and m are zero or 1; and B and B' may be the same or different and represent a leaving group; and, removing any acetyl groups when used as a protecting group Y, a protecting group C or an amino-protecting group in $R^{6'}$, $R^{7'}$ or $R^{8'}$, to obtain a compound of the general formula (I):

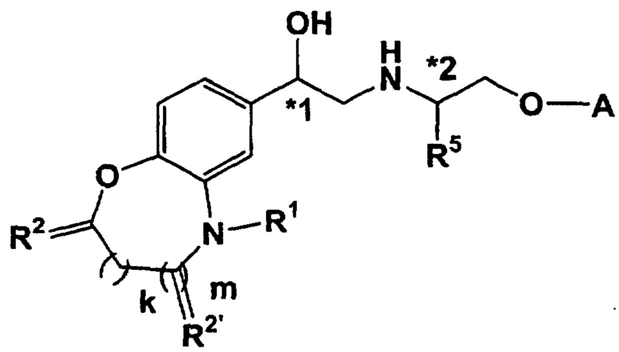

(I)

wherein $R^1$, $R^2$, $R^{2'}$, $R^5$, k, m, A, *1, *2 and *3 have the same meanings as defined above.

The protecting group C is not limited as long as it is a protecting group commonly used as a hydroxyl-protecting group. The protecting group C may be methyl or benzyl which can usually be easily and selectively removed.

The amine-protecting group Y is not limited as long as it is a protecting group commonly used. Examples of the amine-protecting group Y include benzyl, substituted benzyl, benzyloxycarbonyl, substituted benzyloxycarbonyl, tert-butoxycarbonyl, acetyl and trifluoroacetyl which can usually be easily removed.

The deprotecting processes may be sequentially or simultaneously carried out. The deprotecting processes may be preferably carried out in order of removing the protecting group C, the acetyl group as an amino-protecting group, and the amine-protecting group Y. As for the deprotecting condition, when a benzyl group is selected as the protecting group C, it is removed by a hydrogenolysis reaction with a catalyst such as palladium or nickel in a solvent such as methanol. Alternatively, when the protecting group C is benzyl, methyl or the like, it is removed by a treatment with a Lewis acid such as boron tribromide in a solvent such as methylene chloride.

The acetyl-protected amino group can be deprotected by a hydrochloric acid treatment in a solvent such as methanol at room temperature, or by heating with an alkali in a solvent such as water or methanol. The amine-protecting group Y can be removed by a usual method such as a hydrogenolysis reaction with a catalyst such as palladium-carbon in a solvent such as methanol; a treatment with hydrogen bromide/acetic acid; or a hydrochloric acid treatment in dioxane.

The reaction of a compound of the general formula (IV) with a compound of the general formula (V) can be carried out with 1 to 3 equivalents of the latter in the presence of a base in a solvent such as dimethylacetamide, dimethylformamide, acetone or methylene chloride at a temperature of from ice cooling to reflux temperature of the

solvent or 100°C. The base may be potassium carbonate, sodium carbonate, sodium hydroxide, potassium hydroxide, triethylamine or pyridine, and is preferably used at an amount of 1 to 10 equivalents, more preferably 1 to 3 equivalents, with respect to the compound of the general formula (IV).

In the general formula (V), B and B' which may be the same or different represent a leaving group. Specifically, B and B' may be halogen or a lower alkoxy group. Halogen may be fluorine, chlorine, bromine or iodine. The lower alkoxy group may be methoxy, ethoxy, propoxy or tert-butoxy. Examples of specific compounds represented by the general formula (V) include alkylene dihalide such as methylene dibromide, methylene dichloride, ethylene dibromide and triethylene dibromide. In addition, specific compounds represented by the general formula (V) may also be carbonyldiimidazole, thiocarbonyldiimidazole, diethyl carbonate ester, oxalic dichloride, diethyl oxalate ester, ethyl bromoacetate ester or bromoacetic bromide when $R^2$ or $R^{2'}$ is O or S.

Compounds of the general formula (IV) in which A' represents the general formula (II') can be prepared by the process disclosed by the present inventors in WO 97/25311. Such compounds in which A' represents the general formula (III') can be prepared by, for example, the following process. A compound of the general formula (IV) in which $R^1$ represents a hydrogen atom, a methyl group or $SO_2R^3$; $R^3$ represents a lower alkyl group or $NR^4R^{4'}$; $R^4$ and $R^{4'}$ may be the same or different and represent a hydrogen atom, a lower alkyl group or a benzyl group; $R^5$ represents a hydrogen atom or a lower alkyl group; and A' represents the general formula (III'), can be prepared by reacting a compound of the general formula (VI):

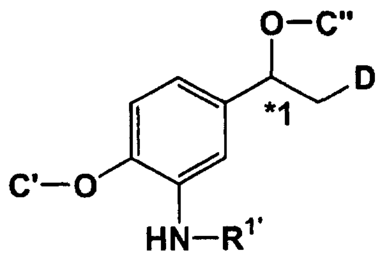

(VI)

wherein C' and C" represent a hydroxyl-protecting group; $R^{1'}$ represents an amino-protecting group, a methyl group or $SO_2R^3$; $R^3$ represents a lower alkyl group or $NR^4R^{4'}$; $R^4$ and $R^{4'}$ may be the same or different and represent a hydrogen atom, a lower alkyl group or a benzyl group; D represents a bromine atom or a chlorine atom; and *1 represents an asymmetric carbon atom,
with a compound of the general formula (VII):

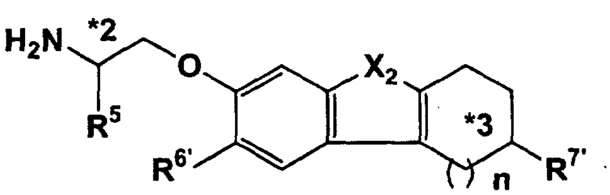

(VII)

wherein $R^5$ represents a hydrogen atom or a lower alkyl group; n is 1 or 2; $X_2$ represents a secondary nitrogen atom, an oxygen atom or a sulfur atom; when n is 1, then one of $R^{6'}$ and $R^{7'}$ represents a hydrogen atom, and the other represents a hydrogen atom, an acetylamino group or a hydroxyl group protected with a protecting group C; when n is 2, then $R^{7'}$ represents a hydrogen atom, and $R^{6'}$ represents a hydrogen atom, an acetylamino group or a hydroxyl group protected with a protecting group C; and, *2 and *3 represent an asymmetric carbon atom when $R^5$ and $R^{7'}$ are not a hydrogen atom, followed by removing the protecting groups C' and C" and the amino-protecting group in $R^{1'}$.

The protecting groups C' and C" are not limited as long as they are a protecting group commonly used as a hydroxyl-protecting group. As a protecting group which can usually be easily and selectively removed, the protecting group C' may be benzyl, tertbutyldimethylsilyl or the like, and the protecting group C" may be triethylsilyl or the like. These hydroxyl-protecting groups can be introduced by known methods. A benzyl group can be introduced to the compound by, for example, adding 1 to 2 moles of benzyl bromide and 1.1 moles of sodium iodide per mole of the compound in the presence of potassium carbonate in a solvent such as dimethylformamide to react them at room temperature. A triethylsilyl group can be introduced to the compound by, for example, reacting with the compound a silylating agent, such as 1.2 to 2 moles of triethylsilyl chloride per mole of the compound in a solvent such as pyridine at a temperature of from 0 to 30°C for 1 to 3 hours.

The amino-protecting group in $R^{1'}$ is not limited as long as it is a protecting group commonly used for protecting aniline. Among such protecting groups, the amino-protecting group is preferably an acetyl group. A process for the acetylation of the compound may comprise a reaction of acetic anhydride with the compound in a solvent such as pyridine. A coupling reaction of a compound of the general formula (VI) with an amine of the formula (VII) is carried out by heating 1 to 1.5 moles of the amine of the formula (VII) per mole of the halide of the formula (VI) in the presence of an amine such as triethylamine or diisopropylethylamine as a proton scavenger in a polar solvent such as dimethylformamide, dimethylacetamide or dimethylsulfoxide at a temperature of from room temperature to 90°C, preferably at 60°C for 5 to 10 hours.

The deprotecting processes may be sequentially or simultaneously carried out. The deprotecting processes may be preferably in order of removing the protecting group C'', the amino-protecting group in $R^{1'}$, and the protecting group C'. As for the deprotecting condition, a benzyl group as the protecting group C' is removed by a hydrogenolysis reaction with a catalyst such as palladium or nickel in a solvent such as methanol. Alternatively, it is removed by a treatment with a Lewis acid such as boron tribromide in a solvent such as methylene chloride. The protecting group C'' such as a triethylsilyl group can be removed by a treatment with acetic acid and 3- to 5-fold moles of tetrabutylammonium fluoride in tetrahydrofuran at room temperature for 0.5 to 5 hours. An acetyl group as the amino-protecting group in $R^{1'}$ can be removed by, for example, a hydrochloric acid treatment at room temperature in a solvent such as methanol, or by heating with an alkali in a solvent such as water or methanol.

A compound of the general formula (VI) can be obtained by reducing a compound of the general formula (VIII):

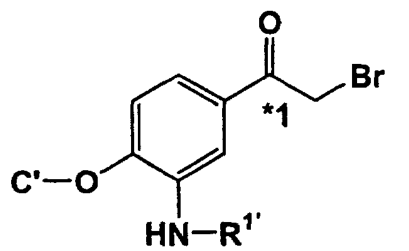

(VIII)

wherein $R^{1'}$ and C' have the same meanings as defined above, according to the following method; when the compound having an iodine atom as the substituent D is to be obtained, replacing the bromine atom with a iodine atom; and then protecting the hydroxyl group.

That is, when the configuration (*1) of the hydroxyl group of a compound of the general formula (VI) is racemic, a compound of the general formula (VIII) is reduced with a reducing agent, such as borane.

In addition, if an optical isomer of either R-form or S-form with respect to *1 of the general formula (VI) is to be obtained, it can be obtained using a chiral auxiliary agent, such as a material represented by the general formula (IX):

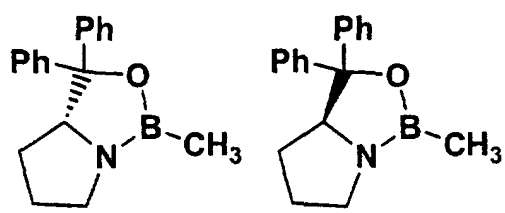

(IX)

That is, it can be obtained by reducing a compound of the general formula (VIII) with borane in the presence of the above chiral auxiliary agent. Said reduction reaction is preferably carried out in a solvent, such as tetrahydrofuran. A process for the preparation of these chiral auxiliary agents and reactions thereof may be carried out in accordance with the literature of E. J. Corey, et al., J. Org. Chem., 56, p. 442 (1991).

When the substitution of iodine atom for the bromine atom (bromine-form) is needed after the reduction of a compound of the general formula (VIII), a compound of interest may be obtained, for example, by heating the reduced compound with an iodinating agent such as 3 to 10 moles of sodium iodide per mole of the bromine-form in a solvent such as acetone at a reflux temperature for 1 to 3 hours.

Next, a compound of the general formula (VI) can be obtained by protecting the hydroxyl group with a protecting group such as a triethylsilyl group according to the above mentioned hydroxyl group-protecting method.

A compound of the general formula (VIII) is a known compound and can be prepared according to the process

indicated in, for example, A. A. Larsen, et al., J. Med. Chem., 10, p. 462 (1967), or C. Kaiser, et al., J. Med. Chem., 17, p. 49 (1974).

A compound of the general formula (VII) can be obtained by reacting a compound of the general formula (X):

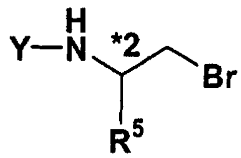

(X)

wherein Y represents an amine-protecting group, and $R^5$ and *2 are as defined above, with a compound of the general formula (XI):

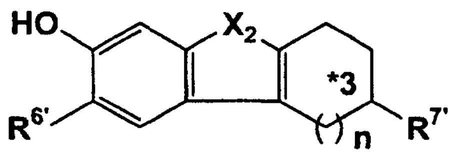

(XI)

wherein n, $X_2$, $R^{6'}$ and $R^{7'}$ are as defined above, followed by removing the amine-protecting group Y. Y means a protecting group of amine and may be any of the above mentioned protecting groups. Deprotection may be carried out by a similar manner.

The reaction of a compound of the general formula (X) with a compound of the general formula (XI) is carried out, for example, in an organic solvent, usually in the presence of a base at a temperature between room temperature and a reflux temperature of the selected solvent. Examples of the solvent include dimethylformamide, dimethylacetamide, acetonitrile, diglyme and tetrahydrofuran. As for the base, potassium carbonate, sodium carbonate, sodium hydroxide, potassium hydroxide, triethylamine, pyridine, sodium hydride, sodium methoxide or the like is used in an amount of 1 to 10 moles per mole of a compound of the formula (XI).

When the reaction slowly proceeds, a compound of the general formula (VII) may be prepared by carrying out the process indicated in Bull. Chem. Soc. Jpn., 55, p. 2504 (1982) or an improved process thereof, followed by removing the amine-protecting group Y. An exemplified process comprises reacting a mixture of an alcohol and 2 to 5 moles of a compound of the general formula (X) and 5 to 10 moles of 40% potassium fluoride/alumina per mole of the alcohol in dimethylformamide or acetonitrile at a temperature of from room temperature to 90°C. According to the improved process, 0.1 to 0.5 equivalent of potassium iodide is also added to the mixture.

A compound of the general formula (X) can be prepared by first protecting amine of commercially available aminoalcohol having $R^5$ and *2 with a protecting group Y, and then brominating the hydroxyl group by a conventional method. Further, if there is an easily available aminobromine-form, the compound may be obtained by protecting amine with a protecting group Y. An exemplified process comprises reacting commercially available 2-bromoethylamine hydrobromate with benzyloxycarbonyl in the presence of triethylamine in methylene chloride with ice cooling.

A compound of the general formula (XI) in which $R^{6'}$ and $R^{7'}$ are a hydrogen atom is a known compound when n is 1. That is, 2-hydroxy-5,6,7,8-tetrahydrocarbazole derivatives of the formula (XI) in which $X_2$ is a secondary nitrogen atom, can be prepared by the process indicated in JP-A-61-57555. 3-Hydroxy-6,7,8,9-tetrahydro-dibenzofuran of the formula (XI) in which $X_2$ is an oxygen atom, can be prepared by the process indicated in DT-2113455 or Erdtman, H. et al., Acta Chem. Scand., 15, p. 1761 (1961). In addition, 3-hydroxy-6,7,8,9-tetrahydrodibenzothiophen of the formula (XI) in which $X_2$ is a sulfur atom, can be prepared by the process indicated in DT-2113455. When n is 2, the compound can be prepared according to the process indicated in the aforementioned patent and literature.

Further, a compound of the general formula (XI) in which n is 1; $R^{6'}$ is a hydrogen atom and $R^{7'}$ is an acetylamino group or a hydroxyl group protected with a protecting group C, can be prepared according to the process indicated in USP-3,959,309 when $X_2$ is a secondary nitrogen atom. When $X_2$ is an oxygen or sulfur atom, the compound can be prepared in accordance with the process indicated in the aforementioned patent or literature from a compound of the general formula (XII) which can be prepared by a conventional method:

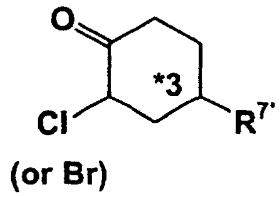

(XII)

wherein $R^{7'}$ and *3 are as defined above.

Further, a compound of the general formula (XI) in which $R^{7'}$ is a hydrogen atom; $R^{6'}$ is an acetylamino group or a hydroxyl group protected with a protecting group C, can be prepared by the following method. For example, a known 2-hydroxy-5,6,7,8-tetrahydrocarbazole derivative in which the hydroxyl group has been benzylated is nitrated (nitro group being introduced on the position of the substituent $R^{6'}$), followed by reducing the nitro group to an amino group. A compound of the general formula (XI) can be obtained by acetylating or diazotizing said amino group, introducing a hydroxyl group, protecting the hydroxyl group with a protecting group C and removing the benzyl group.

The nitrating reaction is carried out according to a conventional process indicated in chemical literatures. An exemplified process comprises nitrating a compound which has been protected with a benzyl group, with the equivalent amount of diluted fuming sulfuric acid in acetic acid at a temperature of from room temperature to 60°C. The reduction reaction of said nitro group can be carried out by a conventional process, such as a process comprising hydrogenating the compound in the presence of platinum oxide as a catalyst in a solvent such as methanol at room temperature or a process comprising reducing the compound with hydrochloric acid in the presence of iron powder or bivalent tin in a solvent such as methanol at a temperature of from room temperature to reflux temperature. The produced amine is acetylated with acetyl chloride in a solvent such as methylene chloride at a temperature of from 0°C to room temperature. Alternatively, said amine is diazotized with sodium nitrite and the resulting diazonium salt is subjected to thermal decomposition in an acidic solution to introduce hydroxyl group, which is then protected with a protecting group C by the aforementioned hydroxyl group-protecting method. Finally, the benzyl group is removed.

Alternatively, a compound of the general formula (IV) may be obtained from a compound of the general formula (XIII):

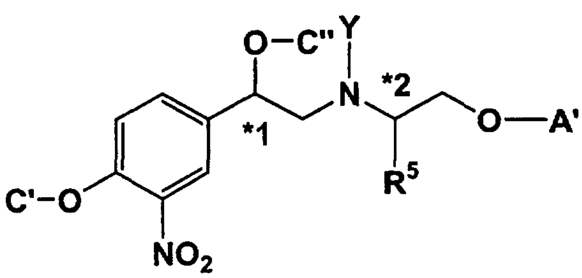

(XIII)

wherein A' represents the general formula (III'); Y represents an amine-protecting group; and C', C", $R^5$, *1 and *2 are as defined above, as an important intermediate.

In this connection, a compound of the general formula (XIII) can be prepared by coupling a compound of the general formula (IV) wherein $NHR^{1'}$ means a nitro group, with a compound of the general formula (VII) followed by protecting amine of the reaction product. The protecting group Y of amine in the general formula (XIII) may be the same with the above mentioned amine-protecting group Y, and may be introduced and removed by a similar manner.

As a process for preparing a compound of the general formula (IV) from a compound of the general formula (XIII) as an intermediate, for example, the following process may be mentioned. That is, a compound of the general formula (XIII) is first reduced (i.e. nitro group of said compound being reduced) to obtain a compound of the general formula (XIV):

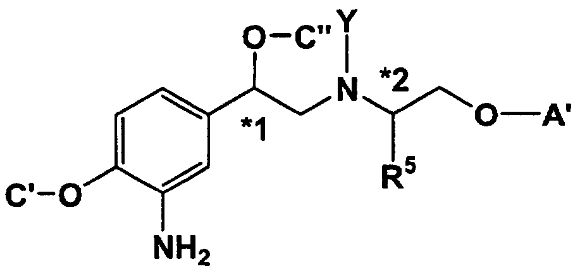

(XIV)

wherein A' represents the general formula (III'); Y represents an amine-protecting group; and C', C", $R^5$, *1 and *2 are as defined above.

This reduction reaction can be carried out by, for example, hydrogenating the compound in the presence of platinum oxide as a catalyst in a solvent such as methanol. Alternatively, it can be carried out in the presence of iron powder or bivalent tin in a solvent such as methanol comprising hydrochloric acid.

A substituent defined as $R^1$ is then introduced by subjecting a compound of the general formula (XIV) to a sulfonation reaction of amine (aniline) according to the method indicated in C. Kaiser, et al., J. Med. Chem., 17, p. 49 (1974) to prepare a compound of the general formula (XV):

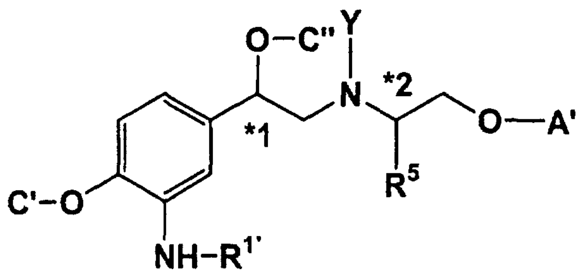

(XV)

wherein A' represents the general formula (III'); Y, C', C", $R^{1'}$, $R^5$, *1 and *2 are as defined above, and the hydroxyl-protecting groups C' and C" are then removed by the aforementioned deprotecting method to obtain a compound of the general formula (IV).

Such a sulfonation reaction may be a reaction of a compound of the general formula (XIV) with $R^3$-substituted sulfonic chloride in a solvent such as pyridine at a temperature of from ice cooling to room temperature.

A variety of compounds of the present invention may be purified, if needed, and such a purification can be usually carried out by a known chromatography (column, flash column, thin layer, or high-performance liquid chromatography) with referring to, for example, $R_f$ values indicated in the present text of specification.

As mentioned above, a compound of the general formula (I) can exist in the form of any of at most eight different isomers. A compound of the general formula (VI) which is a raw material used according to the present invention can provide both pure isomers and racemic mixtures. The reactions described in the above do not alter the stereochemistry involved in such reactions at all.

When a mixture of four or two isomers is obtained, it can be optically resolved by a suitable method such as a method comprising fractionally crystallizing the isomers as acid addition salts with an optically active acid such as camphorsulfonic acid, mandelic acid or substituted mandelic acid. Such a fractional crystallization may be carried out using a suitable solvent, preferably lower alkanol, such as ethanol, isopropanol or a mixture thereof. Optical resolution and purification of the present compounds can provide a preferred medicine which comprises a single isomer having higher activities and thereby has improved efficacy with little side effect.

Salts of a compound of the general formula (I) may be a known salt, and examples thereof include hydrochloride, hydrobromate, sulfate, hydrogensulfate, dihydrogen phosphate, citrate, maleate, tartrate, fumarate, gluconate and methanesulfonate, and acid addition salts with an optically active acid such as camphorsulfonic acid, mandelic acid or substituted mandelic acid. Among them, pharmaceutically acceptable salts are particularly preferred.

When a compound of the general formula (I) is converted into its salt, an acid addition salt of the compound can be obtained by dissolving the compound in alcohol such as methanol or ethanol to which the equivalent amount to several times amount of the acid is added. The acid to be used may be a pharmaceutically acceptable mineral or organic acid, such as hydrochloric acid, hydrobromic acid, sulfuric acid, hydrogensulfate, dihydrogen phosphate, citric acid, maleic acid, tartaric acid, fumaric acid, gluconic acid or methanesulfonic acid.

Heterocyclic compounds of the present invention and pharmaceutically acceptable salts thereof, which have no recognizable toxic effect and little possibility of generating a side effect to increase the heart rate, have usefulness as a medicine, since it has β3-agonist activity and is expected to provide a significant blood concentration due to its excellent permeability through human small intestine epithelium. Therefore, heterocyclic compounds of the present invention and pharmaceutically acceptable salts thereof are expected to be very effective in treating and preventing β3-associated diseases. The term "β3-associated disease" is a generic term directed to diseases which can be improved by agonistic effects mediated by β3-adrenoreceptor. Examples of β3-associated diseases include diabetes, obesity, hyperlipidemia, digestive diseases (preferably dyskinesis of digestive system or ulcer) and depression. According to the present invention, the preferred examples include diabetes, obesity and hyperlipidemia.

A medicine of the present invention is preferably prepared in the form of a pharmaceutical composition by optionally adding a pharmaceutically acceptable carrier to an effective amount of a heterocyclic compound represented by the general formula (I) or a salt thereof. Examples of pharmaceutically acceptable carriers include excipients, binders such as carboxymethylcellulose, disintegrators, lubricants and auxiliaries. When a compound of the present invention is administered to humans, it can be orally administered in the form of tablet, powder, granule, capsule, sugar-coated tablet, solution, syrup or the like. Further, it can be parenterally administered in the form of injection or the like. The dosage administered will vary dependent on the age and weight of the patient and the extent of disease. The daily dosage for an adult is usually 0.01 to 2000 mg, which is singly administered or is divided into several dosages and then administered. The administration period can vary between several weeks and several months and the everyday medication is usually applied. However, the daily dosage and administration period can be increased or decreased from the above ranges dependent on the conditions of patient.

## Examples

The following examples further illustrate this invention but are not intended to limit it in any way.

The thin layer chromatography (TLC) used was Precoated silica gel 60 F254 (mfd. by Merck). After developing with chloroform/methanol (100:1 to 4:1) or ethyl acetate/n-hexane (100:0 to 1:10), the detecting process was carried out with UV (254 nm) irradiation and coloration with ninhydrin. $R_f$ values of TLC were obtained on free amines.

The organic solvents were dried over anhydrous magnesium sulfate or anhydrous sodium sulfate. The column chromatography process was carried out on silica gel (Wako-gel C-200; mfd. by Wako Pure Chemical Industries) and the flash column chromatography process was carried out on silica gel 60 (230-400 mesh; mfd. by Merck). The preparative thin layer chromatography (PTLC) process was carried out on Precoated silica gel 60 F254 20×20 cm, 2 mm (mfd. by Merck). The eluent used was 1:1 chloroform/methanol.

The determination of nuclear magnetic resonance spectrum (NMR) was carried out using Gemini-300 (FT-NMR; mfd. by Varian). As a solvent, unless otherwise specifically mentioned, deuteriumed chloroform was used. Chemical shift was determined using tetramethylsilane (TMS) as the internal standard and is indicated herein in δ(ppm). Coupling constant is indicated herein in J(Hz). Mass spectrum (MS) was determined by the fast atom bombardment mass spectrometry (FAB-MS) with JEOL-JMS-SX102. Data are shown in Table 1.

## Example 1

### (±)-5-[2-[2-(9H-carbazol-2-yloxy)ethylamino]-1-hydroxyethyl]-3-(methylsulfonyl)-3-hydrobenzoxazol-2-one, D-tartrate salt

### A. Synthesis of (±)-N-[5-[2-[benzyl[2-(9H-carbazol-2-yloxy)ethyl]amino]-1-hydroxyethyl]-2-hydroxyphenyl]-methanesulfonamide (Intermediate 1)

To a solution of (±)-N-[5-[2-[2-(9H-carbazol-2-yloxy)-ethylamino]-1-hydroxyethyl]-2-hydroxyphenyl]methanesulfonamide (290 mg; prepared according to the process indicated in WO 97/25311) in dimethylformamide (2 mL) were added potassium carbonate (88 mg; mfd. by KANTO KAGAKU) and benzyl bromide (108.8 mg; mfd. by Wako Pure Chemical Industries) with stirring at room temperature. The resulting mixture was stirred at room temperature for 5 hours. Water was added to the reaction mixture, which was then extracted with ethyl acetate. The organic layer was washed sequentially with water (twice) and saturated brine, and then dried. After the solvent was distilled off under reduced pressure, the residue was purified by column chromatography (1:99 to 2:98 methanol/chloroform) to obtain the title compound (149.8 mg). $R_f$: 0.22 (1:1 ethyl acetate/n-hexane).

B. Synthesis of (±)-5-[2-[benzyl[2-(9H-carbazol-2-yloxy)ethyl]-amino]-1-hydroxyethyll-3-(methylsulfonyl)-3-hydrobenzoxazol-2-one (Intermediate 2)

To a solution of the intermediate 1 (134.1 mg) in dimethylformamide (2 mL) was added 1,1'-carbonyldiimidazole (39.9 mg; mfd. by TOKYO KASEI) with stirring at room temperature. The resulting mixture was stirred at room temperature for 10 minutes. Water was added to the reaction mixture, which was then extracted with ethyl acetate. The organic layer was washed sequentially with water (twice) and saturated brine, and then dried. After the solvent was distilled off under reduced pressure, the residue was purified by column chromatography (1:2 to 1:1 ethyl acetate/n-hexane) to obtain the title compound (109 mg). $R_f$: 0.48 (1:1 ethyl acetate/n-hexane).

C. Synthesis of (±)-5-[2-[2-(9H-carbazol-2-yloxy)ethylamino]-1-hydroxyethyl]-3-(methylsulfonyl)-3-hydrobenzoxazol-2-one, D-tartrate salt

To a solution of the intermediate 2 (109 mg) in dehydrated tetrahydrofuran (5 mL; mfd. by KANTO KAGAKU) was added D-tartaric acid (28.5 mg; mfd. by TOKYO KASEI) under an argon atmosphere. The resulting mixture was stirred at room temperature until the mixture was completely dissolved. After adding 20% palladium hydroxide/activated carbon (50% hydrous material)(20 mg; mfd. by Aldrich) and replacing the atmosphere with hydrogen, the resulting mixture was stirred at room temperature for 19 hours. Dehydrated ethanol (15 mL; mfd. by KANTO KAGAKU) was added to the reaction mixture to dissolve the precipitate, followed by filtering off the catalyst. The filtrate was placed under reduced pressure to distill off the solvent to obtain the title compound (104.1 mg). $R_f$: 0.51 (1:7 methanol/chloroform).

Example 2

(R)-6-[2-[2-(9H-carbazol-2-yloxy)ethylamino]-1-hydroxyethyl]-4-(methylsulfonyl)-2,3-dihydro-1,4-benzoxazine

A. Synthesis of (R)-N-[5-[2-[benzyl[2-(9H-carbazol-2-yloxy)ethyl]amino]-1-hydroxyethyl]-2-hydroxyphenyl]-methanesulfonamide (Intermediate 3)

According to the step A of Example 1, the title compound (31.2 g) was obtained from (R)-N-[5-[2-[2-(9H-carbazol-2-yloxy)-ethylamino]-1-hydroxyethyl]-2-hydroxyphenyl]methanesulfonamide (40 g; prepared according to the process indicated in WO 97/25311), potassium carbonate (18.3 g) and benzyl bromide (11.3 g).
$R_f$: 0.22 (1:1 ethyl acetate/n-hexane);
Retention Time: 31.2 min (S-form: 29.1 min);
Analysis condition:

Column: CHIRALCEL OJ-R (two) (mfd. by Daicel);
Mobile phase: 40/60 0.5 M $NaClO_4/CH_3CN$;
Flow rate: 0.5 mL/min;
Detecting wave length: 254 nm;
Temperature: 40°C.

B. Synthesis of (R)-6-[2-[benzyl[2-(9H-carbazol-2-yloxy)ethyl]-amino)-1-hydroxyethyl]-4-(methylsulfonyl)-2,3-dihydro-1,4-benzoxazine (Intermediate 4)

To a solution of the intermediate 3 (439 mg) in dimethylformamide (5 mL) were added sequentially potassium carbonate (122.3 mg) and 1,2-dibromoethane (166.2 mg; mfd. by TOKYO KASEI) with stirring at room temperature. The resulting mixture was stirred at 60°C for 40 minutes. Water was added to the reaction mixture, which was then extracted with ethyl acetate. The organic layer was washed sequentially with water (twice) and saturated brine, and then dried. After the solvent was distilled off under reduced pressure, the residue was purified by column chromatography (1:2 ethyl acetate/n-hexane) to obtain the title compound (215.6 mg).
$R_f$: 0.74 (1:9 methanol/chloroform).

C. Synthesis of (R)-6-[2-[2-(9H-carbazol-2-yloxy)ethylamino]-1-hydroxyethyll-4-(methylsulfonyl)-2,3-dihydro-1,4-benzoxazine, D-tartrate salt

To a solution of the intermediate 4 (215 mg) in a mixed solvent of tetrahydrofuran (5 mL) and methanol (5 mL) was added 20% palladium hydroxide/activated carbon (50% hydrous material) (40 mg) under an argon atmosphere. After replacing the atmosphere with hydrogen, the mixture was stirred at room temperature for 23 hours. Methanol (10 mL) was added to the reaction mixture to dissolve the precipitate, followed by filtering off the catalyst. The filtrate was placed under reduced pressure to distill off the solvent. The residue was dissolved in a mixed solvent of tetrahydrofuran (15 mL) and methanol (15 mL), to which D-tartaric acid (56 mg) was added to completely dissolve the mixture. The solvent was distilled off to obtain the title compound (226 mg).
$R_f$: 0.19 (1:9 methanol/chloroform).

Example 3

(R)-5-[2-[2-(9H-carbazol-2-yloxy)ethylamino]-1-hydroxyethyll-3-(methylsulfonyl)-2,3-dihydrobenzoxazole, D-tartrate salt

A. Synthesis of (R)-5-[2-[benzyl[2-(9H-carbazol-2-yloxy)-ethyl]amino]-1-hydroxyethyl]-3-(methylsulfonyl)-2,3-dihydrobenzoxazole (Intermediate 5)

According to the step B of Example 2, the title compound (626.3 mg) was obtained from the intermediate 3 (1.09 g), potassium carbonate (304.1 mg) and dibromomethane (382.5 mg; mfd. by nacalai tesque).
$R_f$: 0.68 (1:9 methanol/chloroform).

B. Synthesis of (R)-5-[2-[2-(9H-carbazol-2-yloxy)ethylamino]-1-hydroxyethyl]-3-(methylsulfonyl)-2,3-dihydrobenzoxazole, D-tartrate salt

According to the step C of Example 2, the title compound (643.3 mg) was obtained from the intermediate 5 (611.6 mg), 20% palladium hydroxide/activated carbon (50% hydrous material) (40 mg) and D-tartaric acid (156.1 mg).
$R_f$: 0.24 (1:9 methanol/chloroform).

Example 4

(R)-7-[2-[2-(9H-carbazol-2-yloxy)ethylaminol-1-hydroxyethyl]-5-(methylsulfonyl)-2H,3H,4H-benzo[b]1,4-oxazepine

A. Synthesis of (R)-7-[2-[benzyl[2-(9H-carbazol-2-yloxy)-ethyl]amino]-1-hydroxyethyl]-5-(methylsulfonyl)-2H,3H,4H-benzo[b]1,4-oxazepine (Intermediate 6)

According to the step B of Example 2, the title compound (643 mg) was obtained from the intermediate 3 (600 mg), potassium carbonate (167.2 mg) and 1,3-dibromopropane (244.6 mg; mfd. by nacalai tesque).
$R_f$: 0.78 (1:10 methanol/chloroform).

B. Synthesis of (R)-7-[2-[2-(9H-carbazol-2-yloxy)ethylamino]-1-hydroxyethyl]-5-(methylsulfonyl)-2H,3H,4H-benzo[b]1,4-oxazepine

To a solution of the intermediate 6 (643 mg) in a mixed solvent of tetrahydrofuran (24 mL) and methanol (24 mL) was added 20% palladium hydroxide/activated carbon (50% hydrous material) (303 mg) under an argon atmosphere. After replacing the atmosphere with hydrogen, the mixture was stirred at room temperature for 15 hours. The reaction mixture was filtered to remove the catalyst. The filtrate was placed under reduced pressure and the solvent was distilled off to obtain the title compound (487 mg).
$R_f$: 0.25 (1:10 methanol/chloroform).

Example 5

3-[(dimethylamino)sulfonyl]-5-[2-[2-(9H-carbazol-2-yloxy)-ethylamino]-1-hydroxyethyl]-3-hydrobenzoxazol-2-one, D-tartrate salt

A. Synthesis of (±)-N'-[5-[2-[benzyl[2-(9H-carbazol-2-yloxy)ethyl]amino]-1-hydroxyethyl]-2-hydroxyphenyl]-N,N-dimethylsulfamide (Intermediate 7)

To a solution of (+)-N'-[5-[2-[2-(9H-carbazol-2-yloxy)-ethylamino]-1-hydroxyethyl]-2-hydroxyphenyl]-N,N-dimethylsulfamide (241 mg; prepared according to the process indicated in WO 97/25311) in dehydrated dimethylformamide (2 mL) were added sequentially anhydrous potassium carbonate (68.7 mg; mfd. by KANTO KAGAKU) and

benzyl bromide (85.0 mg; mfd. by Wako Pure Chemical Industries). The resulting mixture was subjected to a reaction/treatment according to the process for preparing the intermediate 1. The crude product was purified by column chromatography (silica gel 60N; mfd. by KANTO KAGAKU; 0:100 to 1:99 methanol/chloroform) to obtain the title compound (155.0 mg). $R_f$: 0.42 (1:9 methanol/chloroform).

B. Synthesis of 3-[(dimethylamino)sulfonyll-5-[2-[benzyl[2-(9H-carbazol-2-yloxy)ethyl]amino]-1-hydroxyethyl]-3-hydrobenzoxazol-2-one (Intermediate 8)

According to the process for preparing the intermediate 2, the intermediate 7 (136.0 mg) was reacted with 1,1'-carbonyldiimidazole (22.6 mg; mfd. by TOKYO KASEI) and the reaction mixture was subjected to a similar treatment/purification to obtain the title compound (136.9 mg). $R_f$: 0.36 (1:1 ethyl acetate/n-hexane).

C. Synthesis of 3-[(dimethylamino)sulfonyl]-5-[2-[2-(9H-carbazol-2-yloxy)ethylamino]-1-hydroxyethyl]-3-hydrobenzoxazol-2-one, D-tartrate salt

According to the step C of Example 1, the intermediate 8 (136.0 mg) and D-tartaric acid (34.0 mg; mfd. by TOKYO KASEI) were dissolved in dehydrated tetrahydrofuran (6.6 mL) and subjected to a hydrogenolysis reaction with 20% palladium hydroxide/activated carbon (22.6 mg; 50% hydrous material; mfd. by N. E. CHEMCAT) under a hydrogen atmosphere at 1 atm at room temperature for 22 hours. The crude product (206 mg) obtained after filtering off the catalyst was crystallized from ethyl acetate/ n-hexane (15 mL; 1:1) to obtain the title compound (130.3 mg). $R_f$: 0.29 (1:7 methanol/chloroform).

Example 6

(R)-[[6-[2-[2-(9H-carbazol-2-yloxy)ethylamino]-1-hydroxyethyl]-(2,3-dihydro-1,4-benzoxazin)-4-yl]sulfonyl] dimethylamine

A. Synthesis of (R)-N'-[5-[2-[benzyl[2-(9H-carbazol-2-yloxy)ethyl]amino]-1-hydroxyethyl]-2-hydroxyphenyl]-N,N-dimethylsulfamide (Intermediate 9)

Similarly to the step A of Example 5 (the synthesis of the intermediate 7), anhydrous potassium carbonate (1.12 g; mfd. by KANTO KAGAKU) and benzyl bromide (1.38 g; mfd. by Wako Pure Chemical Industries) were sequentially added to a solution of (R)-N'-[5-[2-[2-(9H-carbazol-2-yloxy)ethylamino]-1-hydroxyethyl]-2-hydroxyphenyl]-N,N-dimethylsulfamide (4.04 g; prepared according to the process indicated in WO 97/25311) in dehydrated dimethylformamide (25 mL). The resulting mixture was subjected to a reaction/treatment according to the process for preparing the intermediate 1. The crude product was purified by column chromatography (silica gel 60N; mfd. by KANTO KAGAKU; 0:100 to 1:100 methanol/chloroform) to obtain the title compound (3.545 g). $R_f$: 0.42 (1:9 methanol/chloroform);
Retention Time: R-form 38.9 min (S-form: 35.2 min);
Analysis condition:

Column: CHIRALCEL OJ-R (two) (mfd. by Daicel);
Mobile phase: 40/60 0.5 M $NaClO_4/CH_3CN$;
Flow rate: 0.5 mL/min;
Detecting wave length: 254 nm;
Temperature: 40°C.

B. Synthesis of (R)-[[6-[2-[benzyl[2-(9H-carbazol-2-yloxy)ethyl]-amino]-1-hydroxyethyl]-(2,3-dihydro-1,4-benzoxazin)-4-yl]-sulfonyl]dimethylamine (Intermediate 10)

According to the step B of Example 2, anhydrous potassium carbonate (270 mg; mfd. by KANTO KAGAKU) and 1,2-dibromoethane (366.3 mg; mfd. by TOKYO KASEI) were sequentially added to a solution of the intermediate 9 (300 mg) in dehydrated dimethylformamide (2.61 mL) at room temperature which was then stirred at 60°C. After 18 hours, the resulting mixture was diluted with ethyl acetate (100 mL), washed with water (100 mL) three times and then saturated brine (100 mL) and dried over anhydrous sodium sulfate. After the solvent was distilled off under reduced pressure, the residue was dissolved in chloroform (10 mL) and then the solvent was distilled off again to obtain the title compound (380 mg). $R_f$: 0.72 (1:20 methanol/chloroform).

C. Synthesis of (R)-[[6-[2-[2-(9H-carbazol-2-yloxy)ethylamino]-1-hydroxyethyl]-(2,3-dihydro-1,4-benzoxazin)-4-yl] sulfonyl]-dimethylamine

According to the step C of Example 2, the intermediate 10 (330 mg) was dissolved in a mixed solvent of dehydrated tetrahydrofuran (17 mL) and methanol (17 mL) and subjected to a hydrogenolysis reaction with 20% palladium hydroxide/activated carbon (55 mg; 50% hydrous material; mfd. by N. E. CHEMCAT) as a catalyst under a hydrogen atmosphere at 1 atm at room temperature. After 13 hours, additional catalyst (50 mg) was newly added and the reaction was allowed to further proceed for 7.5 hours. The catalyst was filtered off and washed with tetrahydrofuran/ethanol (20 mL; 1:4). The combined filtrate and washing were concentrated to dryness under reduced pressure. The residue was triturated in ethanol (4 mL) and the precipitate filtered out was washed with ethanol (4 mL) and then dried under reduced pressure at 35°C for 14 hours to obtain the title compound (212.3 mg; free form).
$R_f$: 0.11 (1:20 methanol/chloroform).

Example 7

(R)-3-[(dimethylamino)sulfonyl]-5-[2-[2-(9H-carbazol-2-yloxy)ethylamino]-1-hydroxyethyl]-3-hydrobenzoxazol-2-one, hydrochloride

A. Synthesis of (R)-N'-[5-[2-[tert-butoxycarbonyl[2-(9H-carbazol-2-yloxy)ethyl]aminol-1-(triethylsilyloxy)ethyl]-2

benzyloxyphenyl]-N,N-dimethylsulfamide (Intermediate 11)

A solution of di-tert-butyl dicarbonate (2.53 g; mfd. by Peptide Kenkyu) in methylene chloride (20 mL) was added to a solution of (R)-N'-[5-[2-[2-(9H-carbazol-2-yloxy)ethylamino]-1-(triethylsilyloxy)ethyl]-2-benzyloxyphenyl]-N,N-dimethylsulfamide (3.20 g; prepared by the process indicated in WO 97/25311) in methylene chloride (30 mL) at room temperature. The resulting mixture was stirred for 5 hours. The reaction mixture was washed sequentially with water and saturated brine. The organic layer was dried and the solvent was distilled off under reduced pressure. The resulting residue was purified by column chromatography (1:7 to 1:4 ethyl acetate/hexane) to obtain the title compound (3.61 g). $R_f$: 0.66 (1:1 ethyl acetate/n-hexane).

B. Synthesis of (R)-N'-[5-[2-[tert-butoxycarbonyl[2-(9H-carbazol-2-yloxy)ethyl]amino]-1-hydroxyethyl]-2-benzyloxyphenyl]-N,N-dimethylsulfamide (Intermediate 12)

The intermediate 11 (2.89 g) was dissolved in dehydrated tetrahydrofuran (49 mL), to which were added sequentially acetic acid (924.2 mg; mfd. by KANTO KAGAKU) and tetrabutylammonium fluoride/tetrahydrofuran (1 M solution; 9.2 mL; mfd. by TOKYO KASEI) at room temperature. The resulting mixture was stirred for one hour. Additional tetrabutylammonium fluoride/tetrahydrofuran (1 M solution; 5.0 mL) was newly added, followed by stirring for 40 minutes. The reaction mixture was diluted with saturated aqueous sodium bicarbonate (500 mL) and then extracted with ethyl acetate (500 mL). The organic layer was separated and washed with saturated brine (500 mL).

After the organic layer was dried over anhydrous sodium sulfate, the solvent was distilled off to obtain a crude product (3.18 g), which was then purified by column chromatography on silica gel 60N (150 g; spherical and neutral; mfd. by KANTO KAGAKU) to obtain the title compound (2.63 g) from the fraction eluted by 1:1 ethyl acetate/n-hexane.
$R_f$: 0.38 (1:1 ethyl acetate/n-hexane).

C. Synthesis of (R)-N'-[5-[2-[tert-butoxycarbonyl[2-(9H-carbazol-2-yloxy)ethyl]amino]-1-hydroxyethyl]-2-hydroxyphenyl]-N,N-dimethylsulfamide (Intermediate 13)

The intermediate 12 (846 mg) was dissolved in a mixed solvent of dehydrated tetrahydrofuran (41 mL) and methanol (41 mL) and subjected to a hydrogenolysis reaction with 20% palladium hydroxide/activated carbon (500 mg; 50% hydrous material; mfd. by N. E. CHEMCAT) as a catalyst under a hydrogen atmosphere at 1 atm at room temperature for 14 hours. The catalyst was filtered off and the filtrate was placed under reduced pressure to distill the solvent off. The resulting residue (about 750 mg) was purified by column chromatography (silica gel 60N; 50 g; spherical and neutral; mfd. by KANTO KAGAKU) to obtain the title compound (324.5 mg) from the fraction eluted by 2:1 ethyl acetate/n-hexane. $R_f$: 0.25 (1:2 ethyl acetate/n-hexane).

D. Synthesis of (R)-3-[(dimethylamino)sulfonyl]-5-[2-[tert-butoxycarbonyl[2-(9H-carbazol-2-yloxy)ethyl]aminol-1-hydroxyethyl]-3-hydrobenzoxazol-2-one (Intermediate 14)

According to the process indicated in the step B of Example 1 (the synthesis of the intermediate 2), the intermediate 13 (585 mg) was dissolved in dehydrated dimethylacetamide (13 mL), to which were added sequentially 1,1'-carbonyldiimidazole (163 mg; mfd. by TOKYO KASEI) and triethylamine (10.1 mg; mfd. by Wako Pure Chemical Industries) at room temperature. The resulting mixture was stirred under an argon atmosphere for 101 hours. The reaction mixture was diluted with an aqueous semisaturated copper sulfate (II) solution (220 mL), extracted with a mixed solvent of ethyl acetate/n-heptane (300 mL; 2:1), and washed sequentially with water (100 mL) and saturated brine (75 mL).

After the organic layer was dried over anhydrous sodium sulfate, the solvent was distilled off. The resulting residue was subjected to column chromatography purification (silica gel 60N; 35g; mfd. by KANTO KAGAKU) to obtain the title compound (567.3 mg) from the fraction eluted by ethyl acetate.
$R_f$: 0.50 (1:2 ethyl acetate/n-hexane).

E. Synthesis of (R)-3-[(dimethylamino)sulfonyl]-5-[2-[2-(9H-carbazol-2-yloxy)ethylamino]-1-hydroxyethyl]-3-hydrobenzoxazol-2-one, hydrochloride

A 4 N hydrogen chloride/1,4-dioxane solution (30 mL; mfd. by Aldrich) was added to a solution of the intermediate 14 (567.3 mg) in tetrahydrofuran (10 mL) at room temperature, which was then stoppered tightly and stirred for 9 hours. The mixture was diluted with diethyl ether (250 mL) with stirring, and subsequently stirred for 30 minutes. After stopping stirring, the precipitate obtained by filtration was washed with diethyl ether (150 mL) and then dried under reduced pressure at 45°C for 21 hours to obtain the title compound (508.8 mg).
$R_f$: 0.48 (1:7 methanol/chloroform).
Retention Time: R-form 9.3 min (S-form: 7.7 min);
Analysis condition:

Column: CHIRALCEL OJ-R (mfd. by Daicel);
Mobile phase: 50/50 0.5 M $NaClO_4$/$CH_3CN$;
Flow rate: 0.5 mL/min;
Detecting wave length: 254 nm;
Temperature: 40°C.

Example 8

(R)-3-[(dimethylamino)sulfonyl]-5-[2-[2-(9H-carbazol-2-yloxy)ethylamino]-1-hydroxyethyl]-3-hydrobenzoxazol-2-thione, hydrochloride

A. Synthesis of (R)-3-[(dimethylamino)sulfonyl]-5-[2-[tert-butoxycarbonyl[2-(9H-carbazol-2-yloxy)ethyl]amino]-1-hydroxyethyl]-3-hydrobenzoxazol-2-thione (Intermediate 15)

Similarly to the step D of Example 7 (the synthesis of the intermediate 14), 1,1'-thiocarbonyldiimidazole (123.8 mg; mfd. by FLUKA) was added to a solution of the intermediate 13 (394 mg) in dehydrated dimethylacetamide (8.8 mL) with ice cooling. The resulting mixture was stirred at the same temperature for 9 hours. The reaction mixture was diluted with an aqueous semisaturated copper sulfate (II) solution (200 mL), extracted with ethyl acetate (200 mL), and washed sequentially with water (100 mL) and saturated brine (100 mL). After the organic layer was dried over anhydrous sodium sulfate, the solvent was distilled off under reduced pressure. The resulting residue was subjected to column chromatography purification (silica gel 60N; 25g; mfd. by KANTO KAGAKU) to obtain the title compound (433 mg) from the fraction eluted by ethyl acetate.
$R_f$: 0.35 (1:1 ethyl acetate/n-hexane).

B. Synthesis of (R)-3-[(dimethylamino)sulfonyl]-5-[2-[2-(9H-carbazol-2-yloxy)ethylamino]-1-hydroxyethyl]-3-hydrobenzoxazol-2- thione, hydrochloride

Similarly to the step E of Example 7, a 4 N hydrogen chloride/1,4-dioxane solution (21 mL; mfd. by Aldrich) was added to a solution of the intermediate 15 (430 mg) in tetrahydrofuran (7 mL) at room temperature, which was then stoppered tightly and stirred for 14.5 hours. The mixture was diluted with diethyl ether (80 mL) with stirring, and subsequently stirred for 30 minutes. After stopping stirring, the precipitate obtained by filtration was washed with diethyl ether (150 mL) and then dried under reduced pressure at 40°C for 8 hours and then at room temperature for 19.5

hours to obtain the title compound (343.0 mg).
$R_f$: 0.45 (1:7 methanol/chloroform).

Example 9

(R)-5-[2-[2-(9H-carbazol-2-yloxy)ethylamino]-1-hydroxyethyl]-3-(methylsulfonyl)-3-hydrobenzoxazol-2-one, hydrochloride

A. Synthesis of (R)-N-[5-[2-[tert-butoxycarbonyl[2-(9H-carbazol-2-yloxy)ethyl]amino]-1-(triethylsilyloxy)ethyl]-2-(benzyloxy)-phenyl]methanesulfonamide (Intermediate 16)

According to the process for preparing (R)-N-[5-[2-[benzyloxycarbonyl[2-(9H-carbazol-2-yloxy)ethyl]amino]-1-(triethylsilyloxy)ethyl]-2-(benzyloxy)phenyl]methanesulfonamide indicated in WO 97/25311 except that instead of benzyl chloroformate ester, di-tert-butyl dicarbonate (mfd. by Wako Pure Chemical Industries) was used to introduce an amino-protecting group, the title compound (3.61 g) was prepared from (R)-N,N-[2-(9H-carbazol-2-yloxy)ethyl]-[2-(triethylsilyloxy)-2-[3-nitro-4-(benzyloxy)-phenyl]]ethyl]amine.
$R_f$: 0.58 (1:2 ethyl acetate/n-hexane).

B. Synthesis of (R)-N-[5-[2-[tert-butoxycarbonyl[2-(9H-carbazol-2-yloxy)ethyl]amino]-1-hydroxyethyl]-2-hydroxyphenyl]-methanesulfonamide (Intermediate 17)

The intermediate 16 (3.50 g) was dissolved in a mixed solvent of tetrahydrofuran (15 mL) and methanol (15 mL). After adding 10% palladium/activated carbon (1.00 g; mfd. by Merck) under an argon atmosphere and replacing the atmosphere in the reaction system with hydrogen, the resulting mixture was stirred at room temperature for 19 hours. The reaction mixture was filtered and the filtrate was placed under reduced pressure to distil the solvent off. The residue was purified by column chromatography (1:99 to 5:95 methanol/chloroform) to obtain the title compound (2.15g).
$R_f$: 0.33 (1:7 methanol/chloroform).

C. Synthesis of (R)-5-[2-[tert-butoxycarbonyl[2-(9H-carbazol-2-yloxy)ethyl]amino]-1-hydroxyethyl]-3-(methylsulfonyl)-3-hydrobenzoxazol-2-one (Intermediate 18) and (R)-5-[2-[tert-butoxycarbonyl[2-(9H-carbazol-2-yloxy)ethyl]aminol-1-hydroxyethyl]-3-hydrobenzoxazol-2-one (Intermediate 19)

According to the process described in the step D of Example 7, to a solution of the intermediate 17 (100 mg) in dehydrated dimethylacetamide (2.34 mL) were added sequentially 1,1'-carbonyldiimidazole (59 mg; mfd. by TOKYO KASEI) and triethylamine (36.4 mg; mfd. by Wako Pure Chemical Industries) at room temperature, followed by stirring for 20 hours. The reaction mixture was poured into ice-water mixture (20 mL) with stirring and then adjusted to pH 6-7 with 2 N hydrochloric acid. The mixture was slowly warmed to room temperature over 1.25 hours with stirring. The precipitate obtained by filtration was washed with water and then air-dried for 1.2 hours. This was purified by PTLC (two sheets; developed with 2:1 ethyl acetate/n-hexane; mfd. by Merck) to obtain the title compound, the intermediate 18 (48.3 mg) as a compound having lower polarity and the title compound, the intermediate 19 (10.9 mg) as a compound having higher polarity.
The intermediate 18: $R_f$: 0.50 (1:2 ethyl acetate/n-hexane)
The intermediate 19: $R_f$: 0.33 (1:2 ethyl acetate/n-hexane)

D. Synthesis of (R)-5-[2-[2-(9H-carbazol-2-yloxy)ethylamino]-1-hydroxyethyl]-3-(methylsulfonyl)-3-hydrobenzoxazol-2-one, hydrochloride

Similarly to the process indicated in the step E of Example 7, a 4 N hydrogen chloride/1,4-dioxane solution (2.1 mL; mfd. by Aldrich) was added to a solution of the intermediate 18 (48 mg) in tetrahydrofuran (3 mL) at room temperature, which was then stoppered tightly and stirred for 27 hours. The mixture was diluted with diethyl ether (50 mL) with stirring, and subsequently stirred for 14 hours. After stopping stirring, the precipitate obtained by filtration was washed with diethyl ether and then dried under reduced pressure at 40°C for 9.5 hours to obtain the title compound (32.7 mg).
$R_f$: 0.27 (1:10 methanol/chloroform).
Retention Time: R-form 20.9 min (S-form: 19.1 min);
Analysis condition:

Column: CHIRALCEL OJ-R (mfd. by Daicel);
Mobile phase: 65/35 0.5 M $NaClO_4/CH_3CN$;
Flow rate: 0.5 mL/min;
Detecting wave length: 254 nm;
Temperature: 40°C.

Example 10

(R)-5-[2-[2-(9H-carbazol-2-yloxy)ethylamino]-1-hydroxyethyl]-3-hydrobenzoxazol-2-one, hydrochloride

Similarly to the process indicated in the step E of Example 7, a 4 N hydrogen chloride/1,4-dioxane solution (1.5 mL; mfd. by Aldrich) was added to a solution of the intermediate 19 (10.9 mg) in tetrahydrofuran (0.7 mL) at room temperature, which was then stoppered tightly and stirred for 19 hours. The mixture was diluted with diethyl ether (70 mL) with stirring, and subsequently stirred for 1.5 hours. After stopping stirring, the precipitate obtained by filtration was washed with diethyl ether and then dried under reduced pressure at 40°C for 9.5 hours to obtain the title compound (7.3 mg).
$R_f$: 0.08 (1:10 methanol/chloroform).

Example 11

(R)-6-[2-[2-(dibenzothiophen-3-yloxy)ethylamino]-1-hydroxyethyl]-4-(methylsulfonyl)-2,3-dihydro-1,4-benzoxazine, D-tartrate salt

A. Synthesis of (R)-N-[5-[2-[tert-butoxycarbonyl[2-(dibenzothiophen-3-yloxy)ethyl]amino]-1-hydroxyethyl]-2-hydroxyphenyl]methanesulfonamide (Intermediate 20)

Similarly to the synthesis of the intermediate 17, the title compound (326 mg) was prepared according to the process indicated in WO 97/25311.
$R_f$: 0.12 (1:1 ethyl acetate/n-hexane)

B. Synthesis of (R)-6-[2-[tert-butoxycarbonyl[2-(dibenzothiophen-3-yloxy)ethyl]amino]-1-hydroxyethyl]-4-(methylsulfonyl)-2,3-dihydro-1,4-benzoxazine (Intermediate 21)

According to the process indicated in the step B of Example 2, the title compound (330 mg) was prepared from the intermediate 20 (320 mg), potassium carbonate (231.7 mg) and 1,2-dibromoethane (315 mg).
$R_f$: 0.10 (1:2 ethyl acetate/n-hexane).

C. Synthesis of (R)-6-[2-[2-(dibenzothiophen-3-yloxy)ethylamino]-1-hydroxyethyl]-4-(methylsulfonyl)-2,3-dihydro-1,4-benzoxazine, D-tartrate salt

The intermediate 21 (320 mg) was dissolved in a 4 N hydrogen chloride/1,4-dioxane solution (7 mL), which was stirred at room temperature for 2 hours. Ether (35 mL) was added to the reaction mixture. After stirring the mixture at room temperature for 15 minutes, the precipitate obtained by filtration was then purified by column chromatography (0:1 to 1:9 methanol/chloroform) to obtain the title compound (176.8 mg) as a free form. This free form and D-tartaric acid (53.2 mg) were dissolved in tetrahydrofuran (10 mL) and methanol (10 mL) and then the solvent was distilled off to obtain the title compound (230 mg).
$R_f$: 0.32 (1:5 methanol/chloroform).

Test Example 1

Human β3-agonist activities

Human β3-agonist activities were determined using CHO (Chinese hamster ovary) cells transfected with pcDNA3 (mfd. by Invitrogen) to which human β3 gene had been inserted. Human β3 fragment was first obtained from human adipose tissue cDNA (mfd. by Clonetech) by PCR using the primer of β3 (Krief, et al., J. Clin. Invest., 91, pp. 344-349 (1993)). The human β3 fragment obtained was then used as a probe and then the full length human β3 gene was obtained from a human genomic library (mfd. by Clonetech).
The above cells were cultured in a Ham F-12 medium supplemented with 10% fetal bovine serum (mfd. by

Dainippon Pharmaceutical), 400 µg/mL geneticin (Gibco BRL), 100 U/mL penicillin and 100 µg/mL streptomycin. After placing these cells ($5\times10^5$) into a 6-well plate and culturing them for 24 hours, they were allowed to stand on a serum-free Ham F-12 medium for 2 hours. The compound was first dissolved in DMSO, repeatedly diluted by ten times with Ham F-12 supplemented with 1 mM isobutylmethylxanthine and 1 mM ascorbic acid to a final concentration of $10^{-5}$ to $10^{-12}$ M, and then added to the cells.

After the cells were cultured for 30 minutes, the medium was removed followed by addition of 0.5 mL of 1 N NaOH. The medium was allowed to stand for 20 minutes and then 0.5 mL of 1 N acetic acid was added to the medium. The medium was stirred and centrifuged followed by quantitating cAMP with cAMP EIA kit (mfd. by Cayman). With respect to the six compounds among the compounds synthesized in Examples, their intrinsic activities and $ED_{50}$ are shown in the following Table 2. Isoproterenol was purchased from RBI (Research Biochemical International). The results from Table 2 indicate that the compounds of the present invention had activities on human β3. In Table 2, the intrinsic activity (%) appended with an asterisk means a relative value as compared with isoproterenol.

Table 2

| Compound | *Intrinsic Activity (%) | $ED_{50}$ (nM) |
|---|---|---|
| Isoproterenol | 100 | 140 |
| Example 2 | 69 | 12 |
| Example 3 | 70 | 65 |
| Example 4 | 107 | 620 |
| Example 7 | 76 | 100 |
| Example 8 | 99 | 610 |
| Example 10 | 99 | 170 |

Test Example 2

Effects on the heart

The heart was excised from a male guinea pig weighing 180-250 g to prepare a specimen of the right atrium. The specimen was set in an organ bath filled with a Krebs solution which had been aerated with a mixed gas of 5% $CO_2$/95% $O_2$. Each of the present compounds synthesized in Examples was added to the Krebs solution. The automaticity was determined using a isometric transducer (NIHON KOHDEN TB-611T) connected to a polygraph (NIHON KOHDEN MR-6000). The present compounds synthesized in Examples showed higher $ED_{50}$ values for the automaticity as compared with $ED_{50}$ values for β3, and therefore are expected to have selective actions and little induce an increase of the heart rate. That is, the present compounds are expected to have little side effects.

Test Example 3

Permeability through human small intestine epithelium

A model of human small intestine epithelium was prepared according to P. Artursson, Critical Reviews in Therapeutic Drug Carrier Systems, 8, pp. 305-330 (1991), and the following determinations was carried out as an experiment for predicting the orally administered drug-absorbing rate of humans. Human colon adenocarcinoma, Caco-2 cell, when cultured, spontaneously differenciates to form a cell layer consisting of a polar single layer having microvillus similar to small intestine epithelial cell. The presence of tight junction has been found out in the membrane formed. Therefore, the above cell layer can be used as a model system of human small intestine epithelium and is recognized as the simplest system to evaluate the permeability through a membrane of a drug orally administered to human. The specific experimental method is as follows.

1. Culture of Caco-2 cell

Caco-2 (Colon, adenocarcinoma, Human) cells were purchased from ATCC (American Type Culture Collection) and subcultured on DMEM (Dulbecco's modified Eagle medium) supplemented with 10% fetal bovine serum (Gibco BRL) in FALCON T-25 or T-75 culture flask in accordance with P. Artursson, et al., Biochemical and Biophysical Research Communications, 175, No. 3, pp. 880-885 (1991). Before the cells reached confluence, DMEM was removed and the flask was washed with PBS (phosphate buffered saline, pH 7.2, Ca, Mg free (Gibco BRL)) and treated with trypsin/EDTA to peel off the cells, which were then harvested.

After centrifugation (1000 rpm, 5 min), the cells were resuspended in DMEM at $4.4\times10^5$ cells/mL. The cells were seeded into Transwell cell culture chamber (mfd. by Costar) having a collagen-coated membrane filter (3 μm pores, 0.33 $cm^2$ growth area) at 0.15 mL/well ($2\times10^5$ cells/$cm^2$, 24 well). After seeding, DMEM was renewed every other day. After culturing for 18 to 25 days, a membrane formed having a membrane resistance of at least 200 $\Omega \cdot cm^2$ was applied to the drug permeation experiment.

2. Drug permeation experiment

DMEMs in the cup side and base side of Transwell were removed and then replaced with a buffer for the permeation experiment (HBSS (Hanks Balanced Salt Solution, Gibco BRL), 10 mM Mes, pH 6.5 (mfd. by DOJINDO) or 10 mM Hepes, pH 7.4 (mfd. by DOJINDO) containing 25 mM glucose and 0.05% Tween 80). The cup side buffer (0.15 mL) was maintained at pH 6.5 and the base side buffer (0.8 mL) was maintained at pH 7.4. After pre-incubation at 37°C for 10 minutes, the cup side buffer was replaced with the buffer (pH 6.5) containing a drug (20-40 μM). With additionally incubating at 37°C, 80 μL aliquot of the base side buffer was sampled with the passage of time to quantitate the amount of the drug permeated into the base side buffer.

The amount of the drug contained in the base side buffer sampled with the passage of time was divided by the time to calculate the amount of the drug permeated per unit time (sec). It was then divided by the concentration of the drug added and the surface area of the membrane to calculate the permeability coefficient (Papp). That is, the permeability coefficient (Papp) is obtained by the following calculating formula.

$$\mathrm{Papp} = \frac{dQ}{dt} \times \frac{1}{\mathrm{Co} \times \mathrm{A}}$$

wherein dQ/dt is the amount of the drug permeated per unit time (sec); A is the surface area of the membrane; and Co is the concentration of the drug added.

In this connection, the quantitation of the drug was carried out with LC/MS or HPLC (detecting with fluorescence). The conditions of LC/MS and HPLC are as follows.
LC/MS:

- LC condition
  equipment: NANOSPACE SI-1 (mfd. by Shiseido); column: CAPCE LLPAK C18 UG (120 Å, 5 μm, 1.5×150 mm; mfd. by Shiseido); mobile phase: 10 mM ammonium acetate (pH 7.0)/acetonitrile/mixed solvent (gradient 70/30% to 30/70%; for about 30 min); flow rate: 100 μL/min; injection volume: 60 μL.
- MS condition
  equipment: VG QUATTRO II (mfd. by Jasco International); ionization method: electrospray method (ESI); detection: mass corresponding to the molecular weight of each compound + 1 $[M+H]^+$.

HPLC (detecting with fluorescence):

HPLC device: LC-9A (mfd. by Shimadzu); fluorescence detector: RF-535 (mfd. by Shimadzu); excitation wavelength: 305 nm; detecting wavelength: 355 nm; column: Inertsil C8 (4.6×250 mm; mfd. by GL Science); mobile phase: 10 mM $KH_2PO_4$ (pH 4.7)/acetonitrile mixed solvent (isocratic of 65/35% to 50/50%; for about 20 min); flow rate: 0.7 mL/min; injection volume: 10 μL.

The results are shown in the following Table 3. Atenolol (mfd. by Wako Pure Chemical Industries) used as a reference compound is a standard compound having 50% absorption rate through human gastrointestinal tract. Since the permeability coefficients of the present compounds prepared in Examples are comparable to that of atenolol, it is found that the present compounds prepared in Examples are expected to be adequately absorbed by human when orally administered.

Table 3

| | Permeability Coefficient |
|---|---|
| Compound | Papp $\times 10^{-6}$ (cm/s) |
| atenolol | 0.76 |
| Example 2 | 0.93 |
| Example 3 | 1.03 |
| Example 4 | 1.93 |

Table 3 (continued)

| | Permeability Coefficient |
|---|---|
| Compound | Papp × $10^{-6}$ (cm/s) |
| Example 6 | 0.58 |
| Example 7 | 0.78 |
| Example 10 | 0.54 |

Test Example 4

Oral absorptivity by rat

The compound of the present invention was dissolved in a suitable solvent and orally administered to SD rat (CHARLES RIVER) at 10 mg/kg. 30 Minutes, 1 hour and 2 hours after the administration, blood samples were collected and each blood concentration of the compound was determined according to the HPLC method of Test Example 3.
The present compounds were detected in blood immediately after the administration. Therefore, it was found that the present compounds were satisfactorily absorbed and transferred into blood when orally administered.

Test Example 5

Pharmacological effect on a transgenic mice expressing human β3

Since β3 is species specific (Strosberg, et al., Trends Pharmacol. Sci., 17, pp. 373-381 (1996); Strosberg, et al., Annu. Rev. Pharmacol. Toxicol., 37, pp. 421-450 (1997)), pharmacological tests using a transgenic mice expressing human β3 are more effective than those using a normal mice or rat. For example, Ito, et al., showed that human β3 gene into a mouse whose mouse β3 gene had been knocked out, to produce a replacement mice expressing human β3 in its brown fat (Ito, et al., Diabetes, 47, pp. 1464-1471 (1998)).
With a more convenient method, human β3 gene can be expressed in normal mice instead of such knocked out mice. In addition, the best method for directly finding out whether or not a compound has a pharmacological effect on the state of a disease, can be obtained by using genetic obese and diabetic mice with human β3, which is a progeny by crossing human β3 transgenic mice with genetic obese and diabetic mice such as ob/ob, db/db or agouti mice. For example, human β3 gene can be expressed in a tissue which expresses mouse β3 gene by linking mouse β3 promoter to upstream of human β3 gene used in Test Example 1. The method of Hogan, et al. (A Laboratory Manual, 2nd Ed., Cold Spring Harbor Laboratory Press, Cold Spring Harbor, NY) can produce a transgenic mice expressing human β3. The present compounds synthesized in Examples can be evaluated with this model to find the human β3 activities of the orally administered compounds.

Test Example 6

Toxicity test

Each of the present compounds synthesized in Examples was orally administered to 6-week old male mice (CHARLES RIVER JAPAN) at 100 mg/kg, and none were found to be dead. This test indicated a low toxicity of the present compounds.

EFFECT OF THE INVENTION

A compound of the present invention is a novel compound having an activity on human β3. The compound, which has a high permeability coefficient through Caco-2 cells, is certainly expected to be orally absorbed and is also clinically expected to provide a high blood concentration. Therefore, a compound of the present invention is useful as a pharmaceutical composition for treating and preventing β3-associated diseases, such as diabetes, obesity and hyperlipidemia, which composition is particularly suitable for oral administration.

Table 1

| Compound No. | 1H-NMR($CDCl_3$): δ(ppm), J(Hz) | MS(m/z) |
|---|---|---|
| Intermediate 1 | 2.65-2.84(2H, m), 2.84(3H, s), 2.93-2.99(1H, m), 3.01-3.17(1H, m), 3.71(1H, d, J=13.5), 3.96(1H, d, J=13.5), 4.06-4.16(2H, m), 4.61(1H, dd, J=3.3, 10.2), 6.75 (1H, d, J=8.5), 6.82(1H, dd, J=2.2, 8.5), 6.91(1H, d, J=2.2), 6.96(1H, dd, J=2.2, 8.5), 7.16-7.39(9H, m), 7.91(1H, d, J=8.5), 7.96(1H, d, J=7.7), 8.26(1H, br, s) | 546 (MH+) |
| Intermediate 2 | 2.67-2.75(1H, m), 2.89-2.94(1H, m), 3.00-3.20(2H, m), 3.47(3H, s), 3.75 (1H, d, J=13.7), 4.00(1H, d, J=13.7), 4.11-4.16(2H, m), 4.74(1H, dd, J=3.8, 10.4), 6.84 (1H, dd, J=2.2, 8.5), 6.93(1H, d, J=2.2), 7.16-7.41(10H, m), 7.70(1H, d, J=1.6), 7.93(1H, d, J=8.5), 7.97(1H, d, J=8.5), 8.06(1H, br, s) | 572 (MH+) |
| Example 1 | (DMSO-d6; D-tartrate salt): 2.90-2.97(1H, m), 3.05-3.10(1H, m), 3.24-3.28(2H, m), 3.68(3H, s), 4.04(2H, s), 4.26(2H, t, J=5.5), 4.93(1H, dd, J=2.7, 9.3), 6.80(1H, dd, J=2.2, 8.5), 7.00(1H, d, J=2.2), 7.08-7.14(1H, m), 7.26-7.34(2H, m), 7.41-7.47 (2H, m), 7.65(1H, d, J=1.6), 7.97-8.01(2H, m), 11.13(1H, br, s) | 482 (MH+) |
| Intermediate 3 | 2.65-2.84(2H, m), 2.84(3H, s), 2.93-2.99(1H, m), 3.01-3.17(1H, m), 3.71(1H, d, J=13.5), 3.96(1H, d, J=13.5), 4.06-4.16(2H, m), 4.61(1H, dd, J=3.3, 10.2), 6.75(1H, d, J=8.5), 6.82(1H, dd, J=2.2, 8.5), 6.91(1H, d, J=2.2), 6.96(1H, dd, J=2.2, 8.5), 7.16-7.39(9H, m), 7.91(1H, d, J=8.5), 7.96(1H, d, J=7.7), 8.26(1H, br, s) | 546 (MH+) |
| Intermediate 4 | 2.69-2.91(2H, m), 2.91(3H, s), 2.96-3.03(1H, m), 3.09-3.17(1H, m), 3.73(1H, d, J=13.5), 3.85-3.90(2H, m), 4.00(1H, d, J=13.5), 4.09-4.15(2H, m), 4.24-4.27(2H, m), 4.68(1H, dd, J=3.6, 9.9), 6.83(1H, dd, J=2.2, 8.5), 6.89(1H, d, J=8.2), 6.97 (1H, d, J=2.2), 7.06(1H, dd, J=1.9, 8.2), 7.17-7.41(8H, m), 7.69(1H, d, J=1.9), 7.92 (1H, d, J=8.5), 7.97(1H, d, J=7.4), 8.17(1H, br, s) | 572 (MH+) |
| Example 2 | (DMSO-d6; D-tartrate salt): 3.00-3.21(4H, m), 3.13(3H, s), 3.78-3.84(2H, m), 4.16 (2H, s), 4.25-4.30(2H, m), 4.32-4.38(2H, m), 4.88-4.94(1H, m), 6.82(1H, dd, J=2.2, 8.5), 6.95(1H, d, J=8.5), 7.02(1H, d, J=1.9), 7.09-7.14(2H, m), 7.27-7.32(1H, m), 7.42-7.45(1H, m), 7.67(1H, d, J=1.9), 7.99-8.01(2H, m), 11.18(1H, br, s) | 482 (MH+) |
| Intermediate 5 | 2.68-2.76(1H, m), 2.74(3H, s), 2.86-2.92(1H, m), 2.97-3.17(2H, m), 3.73(1H, d, J=13.5), 3.99(1H, d, J=13.5), 4.11-4.16(2H, m), 4.70(1H, dd, J=3.3, 10.2), 5.65-5.70(2H, m), 6.83(1H, dd, J=2.2, 8.5), 6.87(1H, d, J=8.5), 6.95(1H, d, J=2.2), 7.12-7.44(10H, m), 7.92(1H, d, J=8.8), 7.97(1H, d, J=8.0), 8.17(1H, br, s) | 558 (MH+) |
| Example 3 | (DMSO-d6; D-tartrate salt): 2.90-3.40(4H, m), 3.00(3H, s), 4.06(2H, s), 4.27-4.36 (2H, m), 4.91(1H, dd, J=2.5, 9.3), 5.83(2H, s), 6.81(1H, dd, J=2.2, 8.5), 6.99-7.45 (7H, m), 7.97-8.02(2H, m), 11.17(1H, br, s) | 468 (MH+) |
| Intermediate 6 | 1.98-2.08(2H, m), 2.68-3.14(4H, m), 2.91(3H, s), 3.60-3.84(2H, m), 3.92-4.22(6H, m), 4.69(1H, dd, J=3.3, 10.4), 6.82(1H, dd, J=2.2, 8.5), 6.95(1H, d, J=1.9), 7.05 (1H, d, J=8.2), 7.15-7.38(9H, m), 7.47(1H, d, J=1.9), 7.90(1H, d, J=8.5), 7.95(1H, d, J=7.7), 8.32(1H, br, s) | 586 (MH+) |
| Example 4 | (DMSO-d6): 1.98-2.08(2H, m), 2.98-3.76(6H, m), 3.05(3H, s), 3.96-4.08(2H, m), 4.36-4.44(2H, m), 5.00-5.08(1H, m), 6.26-6.30(1H, m), 6.84(1H, dd, J=2.2, 8.5), 7.04(1H, d, J=1.9), 7.09-7.16(2H, m), 7.27-7.36(2H, m), 7.41-7.45(2H, m), 7.98-8.04(2H, m), 9.14(1H, br, s), 11.23(1H, br, s) | 496 (MH+) |
| Intermediate 7 | 2.66(1H, dd, J=12.9, 10.4), 2.70(6H, s), 2.80(1H, dd, J=12.9, 3.3), 2.92-3.03(1H, m), 3.08-3.19(1H, m), 3.70(1H, d, J=13.5), 3.98(1H, d, J=13.5), 4.06-4.18(2H, m), 4.62(1H, dd, J=10.4, 3.3), 6.76(1H, d, J=8.2), 6.83(1H, dd, J=8.5, 2.2), 6.92(1H, d, J=2.2), 6.95(1H, dd, J=8.2, 1.9), 7.19(1H, m), 7.24(1H, d, J=2.2), 7.25-7.41(9H, m), 7.92(1H, d, J=8.5), 7.96(1H, d, J=8.0), 8.24(1H, s) | 575 (MH+) |

Table 1 (continued)

| Compound No. | 1H-NMR($CDCl_3$): δ(ppm), J(Hz) | MS(m/z) |
|---|---|---|
| Intermediate 8 | 2.72(1H, dd, J=12.9, 10.4), 2.91(1H, dd, J=12.9, 3.3), 2.98-3.21(2H, m), 3.10(6H, s), 3.75(1H, d, J=13.5), 4.00(1H, d, J=13.5), 4.13(2H, m), 4.75(1H, dd, J=10.4, 3.3), 6.85(1H, dd, J=8.5, 2.5), 6.94(1H, d, J=2.2), 7.16(1H, m), 7.17-7.24(2H, m), 7.25-7.42(8H, m), 7.65(1H, d, J=1.4), 7.93(1H, d, J=8.5), 7.98(1H, d, J=8.0), 8.08 (1H, br, s) | 601 (MH+) |
| Example 5 | (DMSO-d6; D-tartrate salt): 0.81-0.90(2H, m), 1.25(2H, s), 2.94(1H, dd, J=12.1, 10.0), 3.03(6H, s), 3.08(1H, dd, J=12.1, 3.0), 3.26(2H, m), 4.04(2H, s), 4.26(2H, m), 4.93(1H, dd, J=10.0, 3.0), 6.80(1H, dd, J=8.5, 1.9), 6.99(1H, d, J=1.9), 7.11 (1H, t, J=7.4), 7.26-7.33(2H, m), 7.39-7.46(2H, m), 7.62(1H, s), 7.98(1H, d, J=8.5), 7.99(1H, d, J=8.0), 11.13(1H, s) | 511 (MH+) |
| Intermediate 9 | 2.66(1H, dd, J=12.9, 10.4), 2.70(6H, s), 2.80(1H, dd, J=12.9, 3.3), 2.92-3.03(1H, m), 3.08-3.19(1H, m), 3.70(1H, d, J=13.5), 3.98(1H, d, J=13.5), 4.06-4.18(2H, m), 4.62(1H, dd, J=10.4, 3.3), 6.76(1H, d, J=8.2) , 6.83(1H, dd, J=8.5, 2.2), 6.92(1H, d, J=2.2), 6.95(1H, dd, J=8.2, 1.9), 7.19(1H, m), 7.24(1H, d, J=2.2), 7.257.41(9H, m), 7.92(1H, d, J=8.5), 7.96(1H, d, J=8.0), 8.24(1H, s) | 575 (MH+) |
| Intermediate 10 | 2.71(1H, dd, J=12.9, 10.2), 2.78(6H, s), 2.84(1H, dd, J=12.9, 3.6), 2.92-3.02(1H, m), 3.08-3.18(1H, m), 3.71(1H, d, J=13.7), 3.72-3.78(2H, m), 3.99(1H, d, J=13.7), 4.05-4.17(2H, m), 4.29(2H, t, J=4.7), 4.66(1H, dd, J=10.2, 3.6), 6.83(1H, dd, J=8.5, 2.2), 6.85(1H, d, J=8.2), 6.93(1H, d, J=2.2), 6.98(1H, dd, J=8.5, 2.2), 7.19 (1H, m), 7.23-7.39(8H, m), 7.47(1H, d, J=1.9), 7.91(1H, d, J=8.5), 7.96(1H, d, J=7.7), 8.22(1H, s) | 602 (MH+) |
| Example 6 | (DMSO-d6): 2.83(6H, s), 3.05(1H, m), 3.22(1H, m), 3.46(2H, m), 3.72(2H, m), 4.27(2H, t, J=4.4), 4.38(2H, m), 4.94(1H, m), 6.17(1H, br, s), 6.83(1H, dd, J=8.5, 1.9), 6.93(1H, d, J=8.2), 7.02(1H, d, J=1.9), 7.05-7.16(2H, m), 7.30(1H, m), 7.44 (1H, d, J=8.0), 7.50(1H, d, J=1.7), 7.98-8.04(2H, m), 8.95(1H, br, s), 11.20(1H, s) | 512 (MH+) |
| Intermediate 11 | 0.48-0.57(6H, m), 0.82-0.90(9H, m), 1.47(9H, s), 2.78(6H, s), 3.25-3.70(4H, m), 4.00-4.18(2H, m), 4.85-4.92(1H, m), 5.00-5.09(2H, m), 6.76-6.84(2H, m), 6.91-6.97(2H, m), 7.05-7.64(8H, m), 7.89-7.98(2H, m), 8.20-8.40(1H, m) | 789 (MH+) |
| Intermediate 12 | 1.51(9H, s), 2.77(6H, s), 3.36(1/2H, m), 3.51-3.69(4H, m), 3.83(1/2H, m), 3.96-4.23(1H, m), 4.36(1/2H, m), 4.69(1/2H, m), 4.94-5.13(3H, m), 6.81(1H, m), 6.85-7.02(3H, m), 7.12(1H, m), 7.20(1H, m), 7.28-7.43(7H, m), 7.58(1H, d, J=1.7), 7.92(1H, d, J=8.5), 7.97(1H, d, J=7.7), 8.20-8.41(1H, m) | 675 (MH+) |
| Intermediate 13 | (DMSO-d6) 1.41(9H, s), 2.65(6H, s), 3.24-3.41(3H, m), 3.46-3.62(1H, m), 3.99-4.16(2H, m), 4.65(1H, m), 5.39(1H, br), 6.73(1H, dd, J=8.5, 2.2), 6.81(1H, d, J=8.2), 6.90-ERR-6.99(2H, m), 7.10(1H, m), 7.24-7.32(2H, m), 7.41(1H, d, J=8.0), 7.92-8.01(2H, m), 8.74(1H, br), 9.69(1H, br), 11.06(1H, br, s) | 585 (MH+) |
| Intermediate 14 | 1.51(9H, s), 3.10(6H, s), 3.31(1/4H, m), 3.48-3.76(7/2H, m), 3.90(1/4H, m), 4.16-4.25(5/4H, m), 4.43(1/4H, m), 4.89(1/2H, m), 5.08(1H, m), 6.77-7.00(2H, m), 7.14-7.24(2H, m), 7.25-7.32(2H, m), 7.32-7.42(2H, m), 7.72(1H, s), 7.92(1H, d, J=8.5), 7.96(1H, d, J=7.4), 8.21(1H, s) | 611 (MH+) |
| Example 7 | (DMSO-d6; hydrochloride): 3.05(6H, s), 3.13(1H, m), 3.30(1H, m), 3.49(2H, m), 4.42(2H, m), 5.15(1H, m), 6.42(1H, br, s), 6.84(1H, dd, J=8.5, 2.2), 7.04(1H, d, J=2.2), 7.12(1H, m), 7.26-7.37(2H, m), 7.40-7.51(2H, m), 7.64(1H, s), 7.98-8.05 (2H, m), 9.10(1H, br, s), 9.39(1H, br, s), 11.25(1H, s) | 511 (MH+) |
| Intermediate 15 | 1.51(9H, s), 3.14(6H, s), 3.31(1/2H, m), 3.48-3.77(3H, m), 3.89(1/2H, m), 4.03-4.23(5/4H, m), 4.42(1/4H, m), 4.97(1/2H, m), 5.11(1H, m), 6.76-6.95(2H, m), 7.20(1H, m), 7.26(1H, m), 7.31-7.41(3H, m), 7.85(1H, s), 7.92(1H, d, J=8.5), 7.96 (1H, d, J=7.7), 8.18(1H, s) | 627 (MH+) |

Table 1 (continued)

| Compound No. | 1H-NMR($CDCl_3$): δ(ppm), J(Hz) | MS(m/z) |
|---|---|---|
| Example 8 | (DMSO-d6; hydrochloride): 3.12(6H, s), 3.15(1H, m), 3.34(1H, m), 3.49(2H, m), 4.41(2H, m), 5.19(1H, m), 6.46(1H, br, s), 6.84(1H, dd, J=8.5, 2.2), 7.03(1H, d, J=2.2), 7.12(1H, t, J=7.4), 7.30(1H, m), 7.42-7.48(2H, m), 7.63(1H, d, J=8.5), 7.80(1H, s), 7.99-8.04(2H, m), 9.06(1H, br, s), 9.25(1H, br, s), 11.22(1H, s) | 527 (MH+) |
| Intermediate 16 | 0.48-0.57(6H, m), 0.82-0.90(9H, m), 1.47(9H, s), 2.89(3H, s), 3.25-3.70(4H, m), 4.00-4.18(2H, m), 4.85-4.92(1H, m), 5.00-5.09(2H. m), 6.76-6.84(2H, m), 6.91-6.97(2H, m), 7.05-7.64(8H, m), 7.87-7.98(2H, m), 8.12-8.30(1H, m) | 760 (MH+) |
| Intermediate 17 | (DMSO-d6): 1.39(9H, s), 2.91(3H, s), 3.24-3.62(4H, m), 3.99-4.17(2H, m), 4.67 (1H, m), 5.41(1H, br), 6.74(1H, dd, J=2.2, 8.5), 6.85(1H, d, J=8.0), 6.94-7.30(5H, m), 7.41(1H, d, J=8.0), 7.93-7.99(2H, m), 11.06(1H, br, s) | 556 (MH+) |
| Intermediate 18 | 1.51(9H, s), 3.20-3.99(2H, m), 3.47(3H, s), 3.63(2H, m), 4.0-4.27(1H, m), 4.45 (1/2H, m), 4.93(1/2H, m), 5.09(1H, m), 6.7-6.98(2H, m), 7.16-7.26(3H, m), 7.29-7.42(3H, m), 7.77(1H, s), 7.93(1H, d, J=7.4), 7.97(1H, d, J=8.5), 8.11(1H, s) | 582 (MH+) |
| Intermediate 19 | 1.51(9H, s), 3.30(1/2H, m), 3.60(3H, m), 3.86(1/2H, m), 4.04-4.31(1H, m), 4.40 (1/2H, m), 4.95(1/2H, m), 5.08(1H, m), 6.83(1H, m), 6.92(1H, m), 7.10(1H, m), 7.14-7.26(4H, m), 7.35(1H, m), 7.39(1H, m), 7.94(1H, d, J=8.5), 7.97(1H, d, J=7.4), 8.26(1H, s), 8.76(1H, s) | 504 (MH+) |
| Example 9 | (DMSO-d6; hydrochloride): 3.13(1H, m), 3.32(1H, m), 3.49(2H, m), 3.70(3H, s), 4.40(2H, m), 5.12(1H, m), 6.41(1H, d, J=3.6), 6.84(1H, dd, J=8.5, 2.2), 7.03(1H, d, J=2.2), 7.12(1H, m), 7.27-7.38(2H, m), 7.44(1H, d, J=8.0), 7.50(1H, d, J=8.5), 7.67(1H, d, J=1.7), 8.01(2H, m), 9.03(1H, br, s), 9.21(1H, br, s), 11.21(1H, s) | 482 (MH+) |
| Example 10 | (DMSO-d6; hydrochloride): 3.12(1H, m), 3.32(1H, m), 3.48(2H, m), 4.39(2H, m), 5.03(1H, m), 6.27(1H, m), 6.84(1H, dd, J=8.5, 2.2), 7.02(1H, d, J=2.2), 7.09-7.16 (3H, m), 7.27-7.34(2H, m), 7.44(1H, d, J=8.0), 8.01(2H, m), 8.93(1H, br, s), 9.01 (1H, br, s), 11.19(1H, s), 11.76(1H, s) | 404 (MH+) |
| Intermediate 20 | 1.50(9H, s), 2.94(3H, s), 3.49-3.78(4H, m), 4.11-4.18(2H, m), 4.90-4.98(1H, m), 6.64-6.71(1H, m), 6.86-6.94(1H, m), 7.00-7.46(6H, m), 7.77-7.82(1H, m), 7.99-8.06(2H, m) | 573 (MH+) |
| Intermediate 21 | 1.50(9H, s), 2.95(3H, s), 3.30-3.68(4H, m), 3.76-3.92(2H, m), 4.02-4.16(2H, m), 4.24-4.30(2H, m), 4.93-5.00(1H, m), 6.90-6.96(1H, m), 7.00-7.17(2H, m), 7.22-7.46(3H, m), 7.68-7.72(1H, m), 7.78-7.82(1H, m), 8.00-8.06(2H, m) | 599 (MH+) |
| Example 11 | (DMSO-d6; D-tartrate salt): 1.38(2H, br, s), 3.04-3.28(2H, m), 3.14(3H, s), 3.44-3.50(2H, m), 3.80-3.86(2H, m), 4.25-4.32(4H, m), 4.42-4.48(2H, m), 4.95-5.02(1H, m), 6.96(1H, d, J=8.5), 7.12(1H, dd, J=1.9, 8.5), 7.18(1H, dd, J=2.5, 8.8), 7.42-7.51(2H, m), 7.69-7.70(2H, m), 7.96-7.99(1H, m), 8.25-8.34(2H, m) | 499 (MH+) |

## Claims

**1.** A compound of the general formula (I):

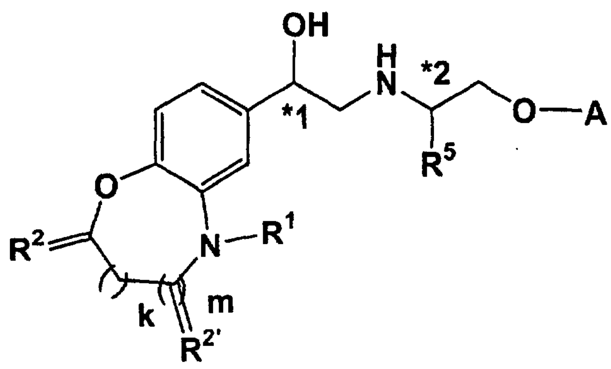

(I)

or a salt thereof,
wherein

$R^1$ represents a hydrogen atom, a methyl group or $SO_2R^3$;
$R^2$ represents O, S or $H_2$;
$R^{2'}$ represents O or $H_2$;
$R^3$ represents a lower alkyl group or $NR^4R^{4'}$;
$R^4$ and $R^{4'}$ may be the same or different and represent a hydrogen atom, a lower alkyl group or a benzyl group;
$R^5$ represents a hydrogen atom or a lower alkyl group;
k and m are zero or 1;
A represents the general formula (II) or (III):

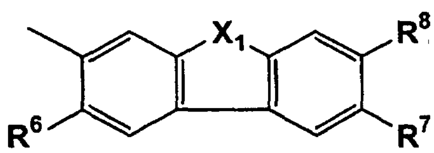

(II)

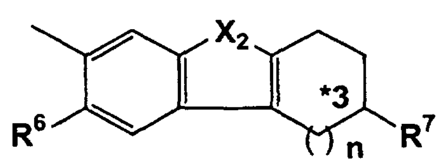

(III)

when A represents the general formula (II),

$X_1$ represents a secondary nitrogen atom, an oxygen atom, a sulfur atom or a methylene group; and
when $X_1$ represents a secondary nitrogen, oxygen or sulfur atom, then $R^8$ represents a hydrogen atom, one of $R^6$ and $R^7$ represents a hydrogen atom, and the other represents a hydrogen atom, an amino group, an acetylamino group or a hydroxyl group; or
when $X_1$ is a methylene group, then $R^6$ and $R^7$ both represent a hydrogen atom and $R^8$ represents a hydrogen atom, an amino group, an acetylamino group or a hydroxyl group; or
when A represents the general formula (III),
n is 1 or 2;
$X_2$ represents a secondary nitrogen atom, an oxygen atom or a sulfur atom; and
when n is 1, then one of $R^6$ and $R^7$ represents a hydrogen atom, and the other represents a hydrogen atom, an amino group, an acetylamino group or a hydroxyl group; or
when n is 2, then $R^7$ represents a hydrogen atom, and $R^6$ represents a hydrogen atom, an amino group, an acetylamino group or a hydroxyl group;

- * 1 represents an asymmetric carbon atom;
- * 2 represents an asymmetric carbon atom when $R^5$ is a lower alkyl group; and
- * 3 represents an asymmetric carbon atom when $R^7$ is an amino, acetylamino or hydroxyl group.

**2.** A compound as claimed in claim 1 having the general formula (I), wherein A represents the general formula (II), or a salt thereof.

**3.** A compound as claimed in claim 1 having the general formula (I), wherein A represents the general formula (III), or a salt thereof.

**4.** A compound as claimed in any one of claims 1 to 3 having the general formula (I), wherein $R^2$ and $R^{2'}$ are $H_2$, or a salt thereof.

**5.** A compound as claimed in any one of claims 1 to 3 having the general formula (I), wherein $R^1$ is $SO_2R^3$, and $R^2$ and $R^{2'}$ are $H_2$, or a salt thereof.

**6.** A compound as claimed in any one of claims 1 to 3 having the general formula (I), wherein $R^2$ represents an oxygen or sulfur atom, and k and m are zero, or a salt thereof.

**7.** A compound as claimed in any one of claims 1 to 3 having the general formula (I), wherein $R^1$ is $SO_2R^3$, and $R^2$ represents an oxygen or sulfur atom, and k and m are zero, or a salt thereof.

**8.** A compound as claimed in any one of claims 1 to 3 having the general formula (I), wherein $R^2$ represents $H_2$, $R^{2'}$ represents an oxygen atom, k is zero, and m is 1, or a salt thereof.

**9.** A compound as claimed in any one of claims 1 to 3 having the general formula (I), wherein $R^2$ represents an oxygen atom, $R^{2'}$ represents an oxygen atom or $H_2$, k is zero, and m is 1, or a salt thereof.

**10.** A compound as claimed in claim 1 or 2, which is selected from the group consisting of:

(R)-2-[2-(9H-carbazol-2-yloxy)ethylamino]-1-(2,3-dihydrobenzoxazol-5-yl)ethan-1-ol;
(R)-5-[2-[2-(9H-carbazol-2-yloxy)ethylamino]-1-hydroxyethyl]-3-(methylsulfonyl)-2,3-dihydrobenzoxazole;
(S)-5-[2-[2-(9H-carbazol-2-yloxy)ethylamino]-1-hydroxyethyl]-3-(methylsulfonyl)-2,3-dihydrobenzoxazole;
5-[2-[2-(9H-carbazol-2-yloxy)ethylamino]-1-hydroxyethyl]-3-(methylsulfonyl)-2,3-dihydrobenzoxazole;
(R)-5-[2-[2-(dibenzothiophen-3-yloxy)ethylamino]-1-hydroxyethyl]-3-(methylsulfonyl)-2,3-dihydrobenzoxazole;
(R)-5-[2-[2-(dibenzofuran-3-yloxy)ethylamino]-1-hydroxyethyl]-3-(methylsulfonyl)-2,3-dihydrobenzoxazole;
(R)-5-[2-[2-(7-acetylaminofluoren-2-yloxy)ethylamino]-1-hydroxyethyl]-3-(methylsulfonyl)-2,3-dihydrobenzoxazole;
(R)-5-[2-[2-(7-aminofluoren-2-yloxy)ethylamino]-1-hydroxyethyl]-3-(methylsulfonyl)-2,3-dihydrobenzoxazole;
(R)-[5-[2-[2-(9H-carbazol-2-yloxy)ethylamino]-1-hydroxyethyl]-(2,3-dihydrobenzoxazol-3-yl)sulfonyl]dimethylamine;
(R)-[5-[2-[2-(dibenzofuran-3-yloxy)ethylamino]-1-hydroxyethyl]-(2,3-dihydrobenzoxazol-3-yl)sulfonyl]dimethylamine;
(R)-[5-[2-[2-(7-acetylaminofluoren-2-yloxy)ethylamino]-1-hydroxyethyl]-(2,3-dihydrobenzoxazol-3-yl)sulfonyl] dimethylamine;
(R)-[5-[2-[2-(7-aminofluoren-2-yloxy)ethylamino]-1-hydroxyethyl]-(2,3-dihydrobenzoxazol-3-yl)sulfonyl] dimethylamine;
(R)-1-[(4H-2,3-dihydro-1,4-benzoxazin)-6-yl]-2-[2-(9H-carbazol-2-yloxy)ethylamino]ethan-1-ol;
(R)-1-[(4-methyl-2,3-dihydro-1,4-benzoxazin)-6-yl]-2-[2-(9H-carbazol-2-yloxy)ethylamino]ethan-1-ol;
(R)-6-[2-[2-(9H-carbazol-2-yloxy)ethylamino]-1-hydroxyethyl]-4-(methylsulfonyl)-2,3-dihydro-1,4-benzoxazine;
(R)-6-[2-[2-(dibenzothiophen-3-yloxy)ethylamino]-1-hydroxyethyl]-4-(methylsulfonyl)-2,3-dihydro-1,4-benzoxazine;
(R)-6-[2-[2-(dibenzofuran-3-yloxy)ethylamino]-1-hydroxyethyl]-4-(methylsulfonyl)-2,3-dihydro-1,4-benzoxazine;
(R)-6-[2-[2-(7-acetylaminofluoren-2-yloxy)ethylamino]-1-hydroxyethyl]-4-(methylsulfonyl)-2,3-dihydro-1,4-benzoxazine;
(R)-6-[2-[2-(7-aminofluoren-2-yloxy) ethylamino]-1-hydroxyethyl]-4-(methylsulfonyl)-2,3-dihydro-1,4-benzoxazine;
(R)-[[6-[2-[2-(9H-carbazol-2-yloxy)ethylamino]-1-hydroxyethyl]-(2,3-dihydro-1,4-benzoxazin)-4-yl]sulfonyl] dimethylamine;
(R)-[[6-[2-[2-(dibenzothiophen-3-yloxy)ethylamino]-1-hydroxyethyl-(2,3-dihydro-1,4-benzoxazin)-4-yl]sulfonyl]-dimethylamine;
(R)-[[6-[2-[2-(dibenzofuran-3-yloxy)ethylamino]-1-hydroxyethyl]-(2,3-dihydro-1,4-benzoxazin)-4-yl]sulfonyl]

dimethylamine;
(R)-[[6-[2-[2-(7-acetylaminofluoren-2-yloxy)ethylamino]-1-hydroxyethyl]-(2,3-dihydro-1,4-benzoxazin)-4-yl] sulfonyl]-dimethylamine;
(R)-[[6-[2-[2-(7-aminofluoren-2-yloxy)ethylamino]-1-hydroxyethyl]-(2,3-dihydro-1,4-benzoxazin)-4-yl]sulfonyl]-dimethylamine;
(R)-7-[2-[2-(9H-carbazol-2-yloxy)ethylamino]-1-hydroxyethyl]-5-(methylsulfonyl)-2H,3H,4H-benzo[b]1,4-oxazepine;
(R)-7-[2-[2-(dibenzothiophen-3-yloxy)ethylamino]-1-hydroxyethyl]-5-(methylsulfonyl)-2H,3H,4H-benzo[b] 1,4-oxazepine;
(R)-7-[2-[2-(dibenzofuran-3-yloxy)ethylamino]-1-hydroxyethyl]-5-(methylsulfonyl)-2H,3H,4H-benzo[b]1,4-oxazepine;
(R)-[[7-[2-[2-(9H-carbazol-2-yloxy)ethylamino]-1-hydroxyethyl]-(2H,3H,4H-benzo[b]1,4-oxazepin-5-yl)]sulfonyl]dimethylamine;
(R)-5-[2-[2-(9H-carbazol-2-yloxy)ethylamino]-1-hydroxyethyl]-3-(methylsulfonyl)-3-hydrobenzoxazol-2-one;
(S)-5-[2-[2-(9H-carbazol-2-yloxy)ethylamino]-1-hydroxyethyl]-3-(methylsulfonyl)-3-hydrobenzoxazol-2-one;
5-[2-[2-(9H-carbazol-2-yloxy)ethylamino]-1-hydroxyethyl]-3-(methylsulfonyl)-3-hydrobenzoxazol-2-one;
(R)-5-[2-[2-(9H-carbazol-2-yloxy)ethylamino]-1-hydroxyethyl]-3-(methylsulfonyl)-3-hydrobenzoxazol-2-thione;
(R)-3-[(dimethylamino)sulfonyl]-5-[2-[2-(9H-carbazol-2-yloxy)ethylamino]-1-hydroxyethyl]-3-hydrobenzoxazol-2-one;
(S)-3-[(dimethylamino)sulfonyl]-5-[2-[2-(9H-carbazol-2-yloxy)ethylamino]-1-hydroxyethyl]-3-hydrobenzoxazol-2-one;
3-[(dimethylamino)sulfonyl]-5-[2-[2-(9H-carbazol-2-yloxy)ethylamino]-1-hydroxyethyl]-3-hydrobenzoxazol-2-one;
(R)-5-[2-[2-(9H-carbazol-2-yloxy)ethylamino]-1-hydroxyethyl]-3-hydrobenzoxazol-2-one;
(R)-5-[2-[2-(9H-carbazol-2-yloxy)ethylamino]-1-hydroxyethyl]-3-methyl-3-hydrobenzoxazol-2-one;
(R)-6-[2-[2-(9H-carbazol-2-yloxy)ethylamino]-1-hydroxyethyl]-2H-1,4-benzoxazin-3(4H)-one;
(R)-6-[2-[2-(9H-carbazol-2-yloxy)ethylamino]-1-hydroxyethyl]-4-methyl-2H-1,4-benzoxazin-3-one;
(R)-6-[2-[2-(9H-carbazol-2-yloxy)ethylamino]-1-hydroxyethyl]-4-(methylsulfonyl)-2H-1,4-benzoxazin-3-one;
(R)-4-[(dimethylamino)sulfonyl]-6-[2-[2-(9H-carbazol-2-yloxy)ethylamino]-1-hydroxyethyl]-2H-1,4-benzoxazin-3-one;
(R)-6-[2-[2-(9H-carbazol-2-yloxy)ethylamino]-1-hydroxyethyl]-3H-1,4-benzoxazin-2(4H)-one;
(R)-6-[2-[2-(9H-carbazol-2-yloxy)ethylamino]-1-hydroxyethyl]-4-methyl-3H-1,4-benzoxazin-2-one;
(R)-6-[2-[2-(9H-carbazol-2-yloxy)ethylamino]-1-hydroxyethyl]-4-(methylsulfonyl)-3H-1,4-benzoxazin-2-one;
and
(R)-4-[(dimethylamino)sulfonyl]-6-[2-[2-(9H-carbazol-2- yloxy)ethylamino]-1-hydroxyethyl]-3H-1,4-benzoxazin-2-one;
or a salt thereof.

**11.** A compound as claimed in claim 1 or 3, which is selected from the group consisting of:

(R)-5-[2-[2-(5,6,7,8-tetrahydro-9H-carbazol-2-yloxy)ethylamino]-1-hydroxyethyl]-3-(methylsulfonyl)-2,3-dihydrobenzoxazole;
(R)-5-[2-[2-(6,7,8,9-tetrahydrodibenzothiophen-3-yloxy)ethylamino]-1-hydroxyethyl]-3-(methylsulfonyl)-2,3-dihydrobenzoxazole;
(R)-5-[2-[2-(5,6,7,8,9,10-hexahydro-cyclohepta[b]indol-2-yloxy)ethylamino]-1-hydroxyethyl]-3-(methylsulfonyl)-2,3-dihydrobenzoxazole;
(R)-[5-[2-[2-(5,6,7,8-tetrahydro-9H-carbazol-2-yloxy)-ethylamino]-1-hydroxyethyl]-(2,3-dihydrobenzoxazol-3-yl)sulfonyl]-dimethylamine;
(R)-[5-[2-[2-(5,6,7,8,9,10-hexahydro-cyclohepta[b]indol-2-yloxy)ethylamino]-1-hydroxyethyl]-(2,3-dihydrobenzoxazol-3-yl)sulfonyl]dimethylamine;
(R)-6-[2-[2-(5,6,7,8-tetrahydro-9H-carbazol-2-yloxy)ethylamino]-1-hydroxyethyl]-4-(methylsulfonyl)-2,3-dihydro-1,4-benzoxazine;
(R)-6-[2-[2-(5,6,7,8,9,10-hexahydro-cyclohepta[b]indol-2-yloxy)ethylamino]-1-hydroxyethyl]-4-(methylsulfonyl)-2,3-dihydro-1,4-benzoxazine;
(R)-[[6-[2-[2-(5,6,7,8-tetrahydrocarbazol-2-yloxy)ethylamino]-1-hydroxyethyl]-(2,3-dihydro-1,4-benzoxazin)-4-yl]sulfonyl]-dimethylamine;
(R)-[[6-[2-[2-(5,6,7,8,9,10-hexahydro-cyclohepta[b]indol-2-yloxy)ethylamino]-1-hydroxyethyl]-(2,3-dihydro-1,4-benzoxazin)-4-yl]sulfonyl]dimethylamine;
(R)-7-[2-[2-(5,6,7,8-tetrahydro-9H-carbazol-2-yloxy)ethylamino]-1-hydroxyethyl]-5-(methylsulfonyl)-2H,3H,4H-benzo[b]1,4-oxazepine;
(R)-[[7-[2-[2-(5,6,7,8-tetrahydro-9H-carbazol-2-yloxy)-ethylamino]-1-hydroxyethyl](2H,3H,4H-benzo[b]1,4-oxazepin-5-yl)]sulfonyl]dimethylamine;
(R)-6-[2-[2-(5,6,7,8-tetrahydro-9H-carbazol-2-yloxy)-ethylamino]-1-hydroxyethyl]-4-(methylsulfonyl)-2H-1,4-benzoxazin-3-one;
(R)-4-[(dimethylamino)sulfonyl]-6-[2-[2-(5,6,7,8-tetrahydro-9H-carbazol-2-yloxy)ethylamino]-1-hydroxyethyl]-2H-1,4-benzoxazin-3-one;
(R)-6-[2-[2-(5,6,7,8-tetrahydro-9H-carbazol-2-yloxy)-ethylamino]-1-hydroxyethyl]-4-(methylsulfonyl)-3H-1,4-benzoxazin-2-one; and
(R)-4-[(dimethylamino)sulfonyl]-6-[2-[2-(5,6,7,8-tetrahydro-9H-carbazol-2-yloxy)ethylamino]-1-hydroxyethyl]-3H-1,4-benzoxazin-2-one;
or a salt thereof.

**12.** A medicine comprising a compound of claim 1 or a salt thereof as an active ingredient.

**13.** A medicine as claimed in claim 12, wherein the medicine is a pharmaceutical composition comprising a compound of claim 1 or a salt thereof as an active ingredient and a pharmaceutically acceptable carrier.

**14.** A medicine as claimed in claim 12, wherein the medicine is for treating or preventing diabetes, obesity or hyperlipidemia.

**15.** A process for the preparation of a compound of the general formula (I):

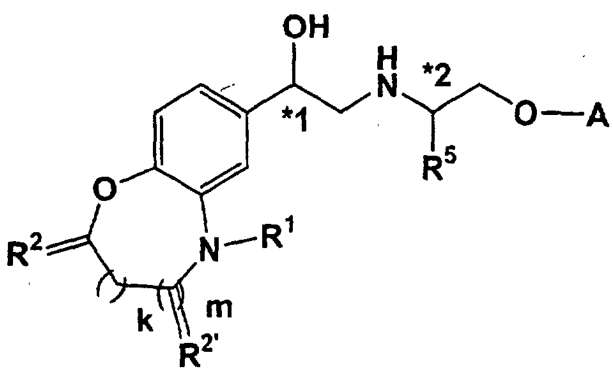

(I)

or a salt thereof,
wherein

$R^1$ represents a hydrogen atom, a methyl group or $SO_2R^3$;
$R^2$ represents O, S or $H_2$;
$R^{2'}$ represents O or $H_2$;
$R^3$ represents a lower alkyl group or $NR^4R^{4'}$ ;
$R^4$ and $R^{4'}$ may be the same or different and represent a hydrogen atom, a lower alkyl group or a benzyl group;
$R^5$ represents a hydrogen atom or a lower alkyl group;
k and m are zero or 1;
A represents the general formula (II) or (III):

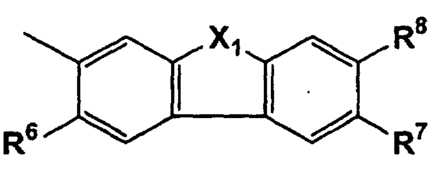

(II)

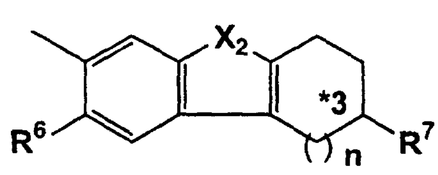

(III)

when A represents the general formula (II),

$X_1$ represents a secondary nitrogen atom, an oxygen atom, a sulfur atom or a methylene group; and when $X_1$ represents a secondary nitrogen, oxygen or sulfur atom, then $R^8$ represents a hydrogen atom, one of $R^6$ and $R^7$ represents a hydrogen atom, and the other represents a hydrogen atom, an amino group, an acetylamino group or a hydroxyl group; or
when $X_1$ is a methylene group, then $R^6$ and $R^7$ both represent a hydrogen atom and $R^8$ represents a hydrogen atom, an amino group, an acetylamino group or a hydroxyl group; or
when A represents the general formula (III),
n is 1 or 2;
$X_2$ represents a secondary nitrogen atom, an oxygen atom or a sulfur atom; and
when n is 1, then one of $R^6$ and $R^7$ represents a hydrogen atom, and the other represents a hydrogen atom, an amino group, an acetylamino group or a hydroxyl group; or
when n is 2, then $R^7$ represents a hydrogen atom, and $R^6$ represents a hydrogen atom, an amino group, an acetylamino group or a hydroxyl group;

* 1 represents an asymmetric carbon atom;
* 2 represents an asymmetric carbon atom when $R^5$ is a lower alkyl group; and
* 3 represents an asymmetric carbon atom when $R^7$ is an amino, acetylamino or hydroxyl group, the process comprising reacting a compound of the general formula (IV) :

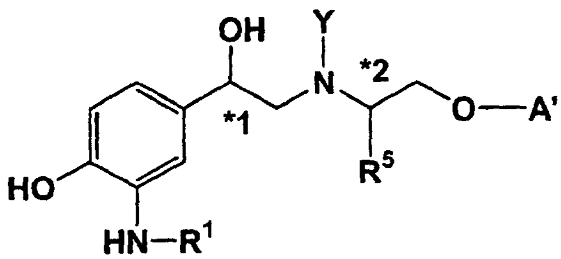
(IV)

wherein

Y represents an amino-protecting group;
A' represents the general formula (II') or (III') :

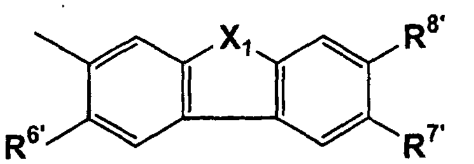
(II')

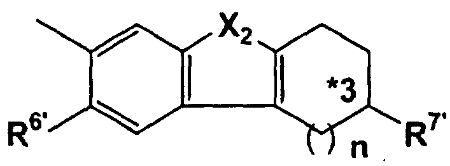
(III')

when A' represents the general formula (II'),
$X_1$ represents a secondary nitrogen atom, an oxygen atom, a sulfur atom or a methylene group; and
when $X_1$ represents a secondary nitrogen, oxygen or sulfur atom, then $R^{8'}$ represents a hydrogen atom, one of $R^{6'}$ and $R^{7'}$ represents a hydrogen atom, and the other represents a hydrogen atom, an acetylamino group or a hydroxyl group protected with a protecting group C; or
when $X_1$ is a methylene group, then $R^{6'}$ and $R^{7'}$ both represent a hydrogen atom and $R^{8'}$ represents a hydrogen atom, an acetylamino group or a hydroxyl group protected with a protecting group C; or when A' represents the general formula (III'),
n is 1 or 2;
$X_2$ represents a secondary nitrogen atom, an oxygen atom or a sulfur atom; and
when n is 1, then one of $R^{6'}$ and $R^{7'}$ represents a hydrogen atom, and the other represents a hydrogen atom, an acetylamino group or a hydroxyl group protected with a protecting group C; or
when n is 2, then $R^{7'}$ represents a hydrogen atom, and $R^{6'}$ represents a hydrogen atom, an acetylamino group

or a hydroxyl group protected with a protecting group C;
*3 represents an asymmetric carbon atom when $R^{7'}$ is an acetylamino group or a hydroxyl group protected with a protecting group C; and
$R^1$, $R^5$, *1 and *2 are as defined above, with a compound of the general formula (V):

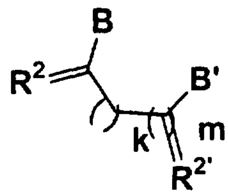

(V)

wherein B and B' may be the same or different and represent a leaving group; and $R^2$, $R^{2'}$, k and m are as defined above, and, removing any acetyl groups when used as a protecting group Y, a protecting group C or an amino-protecting group in $H^{6'}$, $H^{7'}$ or $R^{8'}$ .

## INTERNATIONAL SEARCH REPORT

International application No.
PCT/JP99/06758

A. CLASSIFICATION OF SUBJECT MATTER
Int.Cl[7] C07D263/54, C07D413/12, A61K31/42, A61K31/423, A61K31/538, A61K31/553

According to International Patent Classification (IPC) or to both national classification and IPC

B. FIELDS SEARCHED
Minimum documentation searched (classification system followed by classification symbols)
Int.Cl[7] C07D263/54, C07D413/12, A61K31/42, A61K31/423, A61K31/538, A61K31/553

Documentation searched other than minimum documentation to the extent that such documents are included in the fields searched

Electronic data base consulted during the international search (name of data base and, where practicable, search terms used)
CAPLUS(STN)
REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | WO, 97/25311, A1 (ASAHI KASEI KOGYO KK), 17 July, 1997 (17.07.97) & JP, 9-249623, A1 & EP, 882707, A1 | 1-15 |
| A | US, 4358455, A (Merck and Co.,Inc.), 09 November, 1982 (09.11.82) & JP, 1-265078, A2 & CA, 1215371, A1 | 1-15 |
| A | DE, 2429253, A1 (Boehringer,C.H.,Sohn,Ger.), 15 January, 1976 (15.01.76) (Family: none) | 1-15 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 February, 2000 (24.02.00) | 07 March, 2000 (07.03.00) |
| Name and mailing address of the ISA/ Japanese Patent Office | Authorized officer |
| Facsimile No. | Telephone No. |

Form PCT/ISA/210 (second sheet) (July 1992)